# EUROPÄISCHE PATENTSCHRIFT

(11) **EP 2 370 050 B1**
(45) Veröffentlichungstag und Bekanntmachung des Hinweises auf die Patenterteilung: **01.05.2013**
(21) Anmeldenummer: 09760883.0
(22) Anmeldetag: 30.11.2009
(51) Int. Cl.: A61K 8/49, A61Q 15/00, A61Q 7/02

(54) **NEUE DEODORANTIEN UND ANTITRANSPIRANTIEN MIT HAARWUCHSINHIBIERENDER WIRKUNG**
NOVEL DEODORANTS AND ANTIPERSPIRANTS HAVING HAIR GROWTH INHIBITING EFFECT
NOUVEAUX DÉODORANTS ET ANTI-TRANSPIRANTS PERMETTANT D'INHIBER LA CROISSANCE PILEUSE

(30) Priorität: 01.12.2008 DE 102008059765
(43) Veröffentlichungstag der Anmeldung: 05.10.2011
(73) Patentinhaber: Henkel AG & Co. KGaA, 40589 Düsseldorf (DE)
(72) Erfinder: KROPF, Christian, 40724 Hilden (DE); HUCHEL, Ursula, 50670 Köln (DE); PETERSOHN, Dirk, 50996 Köln (DE); COX, Bruce, Scottsdale AZ 85259 (US); WELSS, Thomas, 40591 Düsseldorf (DE)
(86) Internationale Anmeldenummer: PCT/EP2009/066064
(87) Internationale Veröffentlichungsnummer: WO 2010/063678

(56) Entgegenhaltungen:
- EP-A2- 1 319 392
- WO-A1-93/16717
- WO-A1-2008/025698
- WO-A2-02/15870
- WO-A2-2009/007257
- US-A1- 2007 059 264
- US-B1- 6 218 435

## Beschreibung

Die vorliegende Erfindung betrifft kosmetische und dermatologische Antitranspirant- und Deodorant-Zusammensetzungen, die eine verbesserte Wirkung gegen Haarwuchs aufweisen.

Aus modischen, ästhetischen oder anderen Gründen ist Körperbehaarung oft unerwünscht. Einige Areale, in denen Körperbehaarung unerwünscht ist, sind identisch mit den Körperstellen, in denen Deodorantien und/oder Antitranspirantien angewendet werden, beispielsweise die menschliche Achselhöhle. Es hat daher vereinzelt Vorschläge gegeben, kosmetische Mittel dieser Produktklasse mit Haarwuchshemmern auszustatten, um die Häufigkeit der Enthaarung und/oder Rasur vermindern zu können.

Von besonderem Interesse sind daher kosmetische Wirkstoffe zur Behandlung der Haut, insbesondere der Haut im Achselbereich, die eine Haarwuchs inhibierende Wirkung aufweisen. Kosmetische Zusammensetzungen zur Hautbehandlung im Achselbereich sind üblicherweise Deodorantien und/oder Antitranspirantien; beide Zusammensetzungstypen werden in vielen Zubereitungsformen angeboten, beispielsweise in Stiftform, als Aerosolspray, Pumpspray, flüssige oder gelförmige Roll-on-Applikation, Creme, Lotion, Lösung oder Gel.

Deodorantien und Antitranspirantien, die mindestens einen Haarwuchs inhibierenden Wirkstoff enthalten, sind im Stand der Technik bereits bekannt. US 2007/0059264 offenbart Deodorant- oder Antitranspirant-Zusammensetzungen mit haarwuchsinhibierender Wirkung, die α-Difluoromethylornithine sowie einen Deodorant/Antitranspirant Wirkstoff enthalten. Dennoch besteht nach wie vor das Bedürfnis, den Stand der Technik durch neue Haarwuchs inhibierende Wirkstoffe zu bereichern.

Die Offenlegungsschrift DE 10041482 offenbart so genannte AGEs (Advanced Glycation Endproducts) als kosmetische Wirkstoffe zur Induktion und Intensivierung von Bräunungsmechanismen der menschlichen Haut und die Verwendung einer oder mehrerer Substanzen aus der Gruppe der AGEs und/oder deren Vorläufern zur Steigerung der Melaninsynthese der Haut.

Die Offenlegungsschrift DE 10160966 offenbart die Lehre zur Bereitstellung eines Haarfärbemittels, enthaltend mindestens eine Verbindung aus der Gruppe der AGEs, deren synthetischen Vorstufen und/oder der Lipofuscine und die Verwendung eines solchen Haarfärbemittemittels zur Steigerung der Melaninsynthese in der Haarwurzel und der Einlagerung von Melanin im Haar. Beiden Schriften ist gemeinsam, dass kosmetische Wirkstoffe aus der Gruppe der AGEs zur Steigerung der Melaninsynthese im Haar und/oder der Haut benutzt werden. Ein Hinweis auf andere oder weitergehende biologisch relevante Wirkungen der AGEs sind beiden Dokumenten nicht zu entnehmen.

Überraschenderweise wurde nun gefunden, dass Verbindungen aus der Gruppe der AGEs und/oder deren Vorläufern ausgewählt aus den Verbindungen gemäß Formel 15 und 15a auch als haarwuchsinhibierender Wirkstoff, insbesondere in Deodorantien und Antitranspirantien, verwendet werden können.

Gegenstand der vorliegenden Anmeldung sind daher Deodorantien und/oder Antitranspirantien, enthaltend mindestens eine Verbindung aus der Gruppe der AGEs und/oder deren Vorläufern, ausgewählt aus den Verbindungen gemäß Formel 15 und 15a als als haarwuchsinhibierenden Wirkstoff.

Ein weiterer Gegenstand der vorliegenden Anmeldung ist ein Verfahren zur Verringerung des Haarwuchses, wobei eine Deodorant- oder Antitranspirant-Zusammensetzung, enthaltend mindestens eine Verbindung aus der Gruppe der Advanced Glycation Endproducts (AGEs) und/oder deren Vorläufern, die ausgewählt ist aus den Verbindungen gemäß Formel 15 und 15a, topisch in einer wirksamen Menge auf die Haut, insbesondere auf die Haut im Achselbereich, aufgetragen wird.

Ebenfalls Gegenstand der vorliegenden Anmeldung ist ein Verfahren zur Behandlung von Körpergeruch, wobei eine Deodorant- oder Antitranspirant-Zusammensetzung, enthaltend mindestens eine Verbindung aus der Gruppe der Advanced Glycation Endproducts (AGEs) und/oder deren Vorläufern ausgewählt aus den Verbindungen gemäß Formel 15 und 15a auf die Haut aufgetragen wird.

In einer ersten Ausführungsform betrifft die vorliegende Erfindung kosmetische oder dermatologische Deodorant- oder Antitranspirant-Zusammensetzungen, enthaltend in einem kosmetisch oder dermatologisch verträglichen Träger
a) mindestens einen Deodorant- oder Antitranspirant-Wirkstoff,
b) mindestens eine Verbindung aus der Gruppe der Advanced Glycation Endproducts (AGEs) und/oder deren Vorläufern ausgewählt aus den Verbindungen gemäß Formel 15 und 15a.

Erfindungsgemäß sind unter dem allgemein verwendeten Begriff "Haut" die Haut selbst, die Schleimhaut sowie die Hautanhangsgebilde, sofern sie lebende Zellen umfassen, insbesondere Haarfollikel, Haarwurzel, Haarzwiebel, das ventrale Epithel des Nagelbetts (Lectulus) sowie Talgdrüsen und Schweißdrüsen, zu verstehen. Bevorzugt im Sinne der Erfindung ist unter dem allgemein verwendeten Begriff "Haut" die "menschliche Haut" zu verstehen.

Die Bezeichnung AGE kommt vom englischen Begriff "Advanced Glycation Endproducts". AGEs sind beispielsweise erhältlich gemäß der folgenden (beispielhaften) Reaktionsgleichung:

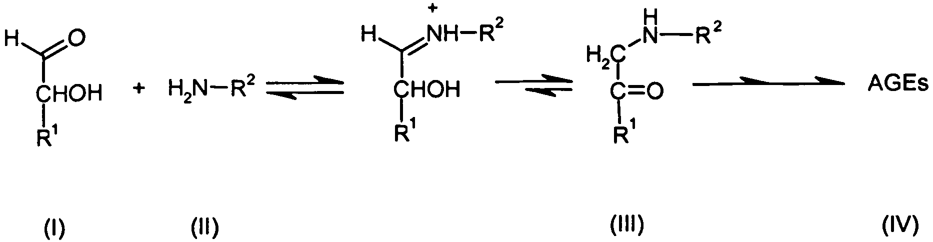

Vorteilhaft sind neben den eigentlichen AGEs auch deren Vorläufer, wie z.B. Amadori- oder Maillard-Produkte (entsprechend (III) in obiger Reaktionsgleichung) bzw. sogenannte EGEs (Early Glycation Endproducts), welche beispielsweise als Zwischenprodukte bei der Umlagerung von den Amadoriprodukten (III) zu den AGEs (IV) erhältlich sind.

Weitere Produkte der Maillard-Reaktion, insbesondere solche die sich auch vom Amadoriprodukt ableiten, wie insbesondere Maltol (Larixin, 3-Hydroxy-2-methyl-4H-pyran-4-on) und 4-Hydroxy-2,5-dimethyl-3(2H)-furanon (Furaneol ®) können ebenfalls vom Begriff "Vorläufer" mit umfasst sein. Unter dem Begriff "Vorläufer" sind im Sinne der vorliegenden Erfindung ausdrücklich nicht die jeweiligen Ausgangsstoffe der Reaktion, also die unumgesetzten Edukte, zu verstehen. Vorteilhafte Ausgangsstoffe (I) tragen eine Aldehydgruppe (CHO) und werden beispielsweise aus der Gruppe der Zucker (Aldosen, z. B. Triosen, Tetrosen, Pentosen, Hexosen und dergleichen wie Glucose, Galactose, Mannose usw.) gewählt. Vorteilhafte Ausgangsstoffe (II) weisen eine freie Aminogruppe auf und werden beispielsweise aus der Gruppe der Aminosäuren und der Gruppe der Peptide mit endständigen Aminogruppen gewählt, insbesondere Lysin, Hydroxylysin, Arginin, Tryptophan und Histidin sind vorteilhaft. Ferner vorteilhaft kann die freie Aminogruppe auch aus N-terminalen Enden von Proteinen und Peptiden stammen, ferner auch vorteilhaft Sphingosin sowie Dihydrosphingosin und deren Homologen mit verschieden langen (ungesättigten) Acylresten.

Weitere Advanced Glycation Endproducts (AGEs) und/oder deren Vorläufer werden durch die Umsetzung einer Aminosäure ausgewählt aus Lysin, Hydroxylysin, Arginin, Tryptophan und/oder Histidin erhalten.

Besonders bevorzugt sind Lysin- und/oder Arginin-basierte Advanced Glycation Endproducts (AGEs) und/oder deren Vorläufer, die durch Umsetzung von Zuckern, vorzugsweise Glukose, Laktose, Galactose und/oder Mannose, insbesondere Glukose und/oder Laktose mit den Aminosäuren Lysin und/oder Arginin, vorzugsweise bei Reaktionstemperaturen von 15°C bis 95°C gebildet werden. Besonders bevorzugt sind die Umsetzungsprodukte, die bei Reaktionen in Temperaturbereichen zwischen 16°C und 35°C, zwischen 36°C und 50°C, zwischen 51°C und 75°C oder zwischen 76°C und 95°C, insbesondere bei 15°C, 20°C, 25°C, 30°C, 35°C, 40°C, 45°C, 50°C, 55°C, 60°C, 65°C, 70°C, 75°C, 80°C, 85°C, 90°C oder 95°C gebildet werden.

Weiterhin bevorzugt sind Lysin- und/oder Arginin-Advanced Glycation Endproducts (AGEs) und/oder deren Vorläufer, die durch Umsetzung von Zuckern, vorzugsweise Glukose, Laktose, Galactose und/oder Mannose, insbesondere Glukose und/oder Laktose mit den Aminosäuren Lysin und/oder Arginin bei Reaktionszeiten von 0,5 h bis 48 h gebildet werden.

Maltosin 15 sowie Pyridosin 15a sind erfindungsgemäße Lysin-basierte AGEs und/oder deren Vorläufer und Produkte der Maillard-Reaktion von Lysin A mit Zuckern.

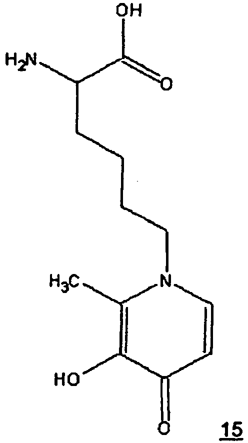

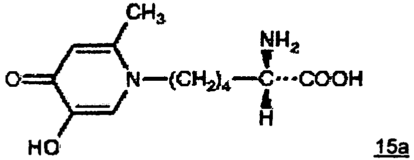

Eine bevorzugte Ausführungsform der Erfindung ist die Verwendung einer erfindungsgemäßen kosmetischen Zusammensetzung mit einem Anteil einer Verbindung oder einer Mischung verschiedener Verbindungen aus der Gruppe AGEs und/oder deren Vorläufern ausgewählt aus den Verbindungen gemäß Formel 15 und 15a von 0,00001 bis 15 Gew.-%, vorzugsweise von 0,0001 bis 5 Gew.-%, besonders bevorzugt von 0,001 bis 1 Gew.-%, jeweils bezogen auf die Gesamtzusammensetzung.

Besonders bevorzugte erfindungsgemäße Deodorant- oder Antitranspirant-Zusammensetzungen enthalten - bezogen auf das Gewicht der anwendungsbereiten Zusammensetzung - 0,00001 bis 15 Gew.-%, vorzugsweise 0,0001 bis 10 Gew.-%, besonders bevorzugt 0,001 bis 5 Gew.-%, weiter bevorzugt 0,01 bis 4 Gew.-% und insbesondere 0,1 bis 2 Gel.-% Advanced Glycation Endproducts (AGEs) und/oder deren Vorläufer ausgewählt aus den Verbindungen gemäß Formel 15 und 15a

Ein besonders bevorzugtes AGE ist Maltosin. Erfindungsgemäß äußerst bevorzugte Deodorant- oder Antitranspirant-Zusammensetzungen enthalten - bezogen auf das Gewicht der anwendungsbereiten Zusammensetzung - 0,0001 bis 15 Gew.-%, vorzugsweise 0,001 bis 12,6 Gew.-%, besonders bevorzugt 0,01 bis 10 Gel.-%, weiter bevorzugt 0,1 bis 5 Gel.-% und insbesondere 0,25 bis 2,5 Gew.-% Maltosin (Formel 15).

### Antitranspirant-Wirkstoffe

Erfindungsgemäß bevorzugte Antitranspirant-Wirkstoffe sind ausgewählt aus den Aluminiumchlorhydraten, insbesondere den Aluminiumchlorhydralen mit der allgemeinen Formel [Al₂(OH)₅Cl · 2-3 H₂O]ₙ, die in nicht-aktivierter oder in aktivierter (depolymerisierter) Form vorliegen können, weiterhin Aluminiumsesquichlorhydrat, Aluminiumchlorhydrex-Propylenglycol (PG) oder -Polyethylenglycol (PEG). Aluminiumesquichlorhydrex-PG oder -PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ · 12 H₂O, Alaun), Aluminiumundecylenoylkollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind. Die Antitranspirant-Wirkstoffe können als wässrige Lösungen eingesetzt werden.

Besonders bevorzugte erfindungsgemäße Deodorant- oder Antitranspirant-Zusammensetzungen sind dadurch gekennzeichnet, dass der mindestens eine Antitranspirant-Wirkstoff in einer Gesamtmenge von 3 - 25 Gel.-%, vorzugsweise 5 - 22 Gel.-% und insbesondere 10 - 20 Gel.-%, enthalten ist. bezogen auf das Gesamtgewicht der Aktivsubstanz in der Gesamtzusammensetzung. In einer besonders bevorzugten Ausführungsform enthält die Zusammensetzung ein adstringierendes Aluminiumsalz, insbesondere Aluminiumchlorohydrat, das beispielsweise pulverförmig als Micro Dry^{®} Ultrafine von Reheis, in Form einer wässrigen Lösung als Locron^{®} L von Clariant, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten, das ebenfalls besonders bevorzugt ist. Auch die Verwendung von Aluminium-Zirkonium-Tetrachlorohydrex-Glycin-Komplexen, die beispielsweise von Reheis unter der Bezeichnung Rezal^{®} 36G im Handel sind, kann erfindungsgemäß besonders bevorzugt sein.

Die erfindungsgemäßen Zusammensetzungen können in einer weiteren besonders bevorzugten Ausführungsform sowohl mindestens einen Deodorant- als auch mindestens einen Antitranspirant-Wirkstoff enthalten.

### Deodorant-Wirkstoffe

Erfindungsgemäß bevorzugte Deodorant-Wirkstoffe sind Geruchsabsorber, desodorierend wirkende Ionenaustauscher, keimhemmende Mittel, präbiotisch wirksame Komponenten sowie Enzyminhibitoren oder, besonders bevorzugt, Kombinationen der genannten Wirkstoffe.

Silicate dienen als Geruchsabsorber, die auch gleichzeitig die rheologischen Eigenschaften der erfindungsgemäßen Zusammensetzung vorteilhaft unterstützen. Zu den erfindungsgemäß besonders bevorzugten Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere bevorzugte Geruchsabsorber sind beispielsweise Zeolithe, Zinkricinoleat, Cyclodextrine, bestimmte Metalloxide, wie z. B. Aluminiumoxid, sowie Chlorophyll. Sie werden bevorzugt in einer Menge von 0,1 - 10 Gew.-%, besonders bevorzugt 0,5 - 7 Gew.-% und außerordentlich bevorzugt 1 - 5 Gew.-%, jeweils bezogen auf die Gesamtzusammensetzung, eingesetzt.

Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen,-Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzethoniumchlorid. Weiterhin sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆- C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.

Weitere bevorzugte Deodorant-Wirkstoffe sind ausgewählt aus sogenannten präbiotisch wirksamen Komponenten, worunter efindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit einbezogen sind hier Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.

Weitere bevorzugte Deodorant-Wirkstoffe sind ausgewählt aus den keimhemmend wirkenden Parfümölen und den Deosafe^{®}-Parfümölen, die von der Firma Symrise, vormals Haarmann und Reimer, erhältlich sind.

Zu den Enzyminhibitoren gehören Stoffe, die die für die Schweißzersetzung verantwortlichen Enzyme, insbesondere die Arylsulfatase, β-Glucuronidase, Aminoacylase, Esterasen, Lipasen und/oder Lipoxigenase, hemmen, z. B. Trialkylcitronensäureester, insbesondere Triethylcitrat, oder Zinkglycinat.

Bevorzugte erfindungsgemäße Deodorant- oder Antitranspirant-Zusammensetzungen sind dadurch gekennzeichnet, dass der mindestens eine Deodorant-Wirkstoff ausgewählt ist aus Arylsulfatase-Inhibitoren, β-Glucuronidase-Inhibitoren, Aminoacylase-Inhibitoren, Esterase-Inhibitoren, Lipase-Inhibitoren und Lipoxigenase-Inhibitoren, α-Monoalkylglycerinethern mit einem verzweigten oder linearen gesättigten oder ungesättigten, gegebenenfalls hydroxylierten C₆ - C₂₂-Alkylrest, insbesondere α-(2-Ethylhexyl)glycerinether, Phenoxyethanol, keimhemmend wirkenden Parfümölen, Deosafe^{®}-Parfümölen (Deosafe^{®} ist ein eingetragenes Warenzeichen der Firma Symrise, vormals Haarmann & Reimer), präbiotisch wirksamen Komponenten, Trialkylcitronensäureestern, insbesondere Triethylcitrat, Wirkstoffen, die die Zahl der an der Geruchsbildung beteiligten Hautkeime aus der Gruppe der Staphylokokken, Corynebakterien, Anaerokokken und Mikrokokken reduzieren bzw. deren Wachstum hemmen, Zinkverbindungen, insbesondere Zinkphenolsulfonat und Zinkricinoleat, Organohalogenverbindungen, insbesondere Triclosan, Chlorhexidin, Chlorhexidingluconat und Benzalkoniumhalogeniden, quartären Ammoniumverbindungen, insbesondere Cetylpyridiniumchlorid, Geruchsabsorbern, insbesondere Silikaten und Zeolithen, Natriumbicarbonat, Lantibiotika, sowie Mischungen der vorgenannten Substanzen.

Weitere bevorzugte erfindungsgemäße Deodorant- oder Antitranspirant-Zusammensetzungen sind dadurch gekennzeichnet, dass der mindestens eine Deodorant-Wirkstoff in einer Gesamtmenge von 0,1 - 10 Gew.-%, bevorzugt 0,2 - 7 Gew.-%, besonders bevorzugt 0,3 - 5 Gew.-% und außerordentlich bevorzugt 0,4 - 1,0 Gew.-%, jeweils bezogen auf das Gesamtgewicht der Aktivsubstanz des Deodorant-Wirkstoffs oder der Deodorant-Wirkstoffe in der Gesamtzusammensetzung, enthalten ist.

Weitere bevorzugte Ausführungsformen der Erfindung sind dadurch gekennzeichnet, dass der kosmetisch oder dermatologisch verträgliche Träger als Puder, in Stiftform, als Aerosolspray, Pumpspray, flüssige oder gelförmige Roll-on-Applikation, Creme, Lotion, Lösung, Gel oder auf einem Substrat aufgebracht vorliegt.

Deodorant- oder Antitranspirant-Stifte können in gelierter Form, auf wasserfreier Basis, auf Basis einer W/O-Emulsion, auf Basis einer O/W-Emulsion, auf Basis einer Wasser-Öl-Mehrfach-Emulsion, auf Basis einer Nanoemulsion und auf Basis einer Mikroemulsion vorliegen, wobei die Ölphase mindestens eine Siliconkomponente enthalten oder aus mindestens einer Siliconkomponente bestehen kann. Weiterhin können die erfindungsgemäßen Zusammensetzungen, die als Deodorant- oder Antitranspirant-Stifte formuliert sind, auf wasserfreier Fettbasis, auf Basis einer Polyol-in-Öl-Emulsion, auf Basis einer Öl-in-Polyol-Emulsion, auf Basis einer Polyol-Öl-Mehrfach-Emulsion, auf Basis einer Nanoemulsion und auf Basis einer Mikroemulsion vorliegen, wobei die Polyolphase wasserfrei sein oder nur einen geringen Wassergehalt aufweisen kann. Gelstifte können auf der Basis von Fettsäureseifen, Dibenzylidensorbitol, N-Acylaminosäureamiden, 12-Hydroxystearinsäure, Polyamiden, Polyamidderivaten, Polysacchariden wie Xanthan, Polyglucomannanen, Guar, Konjak, Cellulosen oder Stärken, Polyacrylaten, Polyacrylatderivaten und anderen Gelbildnem formuliert werden.

Aerosolsprays, Pumpsprays, Roll-on-Applikationen und Cremes können als Wasser-in-Öl-Emulsion, Öl-in-Wasser-Emulsion, Siliconöl-in-Wasser-Emulsion, Wasser-in-Öl-Mikroemulsion, Öl-in-Wasser-Mikroemulsion, Siliconöl-in-Wasser-Mikroemulsion, Polyol-in-Öl-Emulsion, Öl-in-Polyol-Emulsion, Polyol-Öl-Mehrfach-Emulsion, wasserfreie Suspension, bevorzugt wasserfreie ölbasierte Suspension, alkoholische Lösung, insbesondere ethanolische Lösung, hydroalkoholische Lösung, insbesondere Lösungen mit mehr als 50 Gew.-% eines Wasser-Ethanol-Gemisches, glycolische Lösung, insbesondere als Lösung in Propylenglycol, Glycerin, Dipropylenglycol und (unter Normalbedingungen) flüssigen Polyethylenglycolen, hydroglycolische Lösung, Polyol-Lösung, Wasser-Polyol-Lösung, wässriges Gel, Lipogel und als Öl vorliegen. Alle genannten Zusammensetzungen können verdickt sein, beispielsweise auf der Basis von Fettsäureseifen, Dibenzylidensorbitol, N-Acylaminosäureamiden, 12-Hydroxystearinsäure, Polyacrylaten vom Carbomer- und Carbopol-Typ, Polyacrylamiden und Polysacchariden, die chemisch und/oder physikalisch modifiziert sein können.

Die Zusammensetzungen können transparent, translucent oder opak sein.

### Lipid- oder Wachsmatrix

Sofern die erfindungsgemäßen Zusammensetzungen in Form eines Stiftes vorliegen, enthalten sie bevorzugt eine Lipid- oder Wachsmatrix, umfassend mindestens eine Lipid- oder Wachskomponente mit einem Schmelzpunkt > 50°C.

Generell sind Wachse von fester bis brüchig harter Konsistenz, grob bis feinkristallin, durchscheinend bis opak, jedoch nicht glasartig, und schmelzen oberhalb von 50 °C ohne Zersetzung. Sie sind schon wenig oberhalb des Schmelzpunktes niedrigviskos und zeigen eine stark temperaturabhängige Konsistenz und Löslichkeit.

Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse und Polyethylenglycolwachse, C₂₀-C₄₀-Dialkylester von Dimersäuren, C₃₀-₅₀-Alkylbienenwachs sowie Alkyl- und Alkylarylester von Dimerfettsäuren.

Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäße Zusammensetzung als besonders vorteilhaft erwiesen, weil sie der Antitranspirantzubereitung ausgezeichnete sensorische Eigenschaften und dem Stift insgesamt eine hohe Stabilität verleihen. Die Ester setzen sich aus gesättigten verzweigten oder unverzweigten Monocarbonsäuren und gesättigten verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten verzweigten oder unverzweigten Alkoholen sind erfindungsgemäß einsetzbar, sofern die Wachskomponente einen Schmelzpunkt > 50°C hat. Besonders bevorzugt ist, die Wachskomponenten zu wählen aus der Gruppe der Ester aus gesättigten verzweigten oder unverzweigten Alkancarbonsäuren einer Kettenlänge von 12 bis 24 C-Atomen und den gesättigten verzweigten oder unverzweigten Alkoholen einer Kettenlänge von 16 bis 50 C-Atomen, die einen Schmelzpunkt > 50°C haben.

Insbesondere können als Wachskomponente C₁₆₋₃₆-Alkylstearate und C₁₈₋₃₈-Alkylhydroxystearoylstearate, C₂₀₋₄₀-Alkylerucate sowie Cetearylbehenat vorteilhaft sein. Das Wachs oder die Wachskomponenten weisen einen Schmelzpunkt > 50°C, bevorzugt > 60°C, auf.

-Eine besonders bevorzugte Ausführungsform der Erfindung enthält als Wachskomponente ein C₂₀-C₄₀-Alkylstearat. Dieser Ester ist unter den Namen Kesterwachs^{®} K82H oder Kesterwachs^{®} K80H bekannt und wird von Koster Keunen Inc. vertrieben. Eine weitere besonders bevorzugte Ausführungsform der Erfindung enthält als Wachskomponente Cetearylbehenat, d. h. Mischungen aus Cetylbehenat und Stearylbehenat. Dieser Ester ist unter dem Namen Kesterwachs^{®} K62 bekannt und wird von Koster Keunen Inc. vertrieben.

Weitere bevorzugte Lipid- oder Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax^{®} HGL-C). Erfindungsgemäß ist als Wachskomponente hydriertes Rizinusöl, erhältlich z.B. als Handelsprodukt Cutina^{®} HR, besonders bevorzugt.

Weitere bevorzugte Lipid- oder Wachskomponenten mit einem Schmelzpunkt > 50°C sind die gesättigten linearen C₁₄ - C₃₆-Carbonsäuren, insbesondere Myristinsäure, Palmitinsäure, Stearinsäure und Behensäure sowie Mischungen dieser Verbindungen, z. B. Syncrowax^{®} AW 1C (C₁₈ - C₃₆-Fettsäuren) oder Cutina^{®} FS 45 (Palmitin- und Stearinsäure).

Bevorzugte erfindungsgemäße Deodorant- oder Antitranspirant-Stifte sind dadurch gekennzeichnet, dass die Lipid- oder Wachskomponente a) ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄ - C₃₆-Carbonsäuren sowie Mischungen der vorgenannten Substanzen. Besonders bevorzugte Lipid- oder Wachskomponenten-Mischungen a) sind ausgewählt aus Mischungen von Cetylbehenat, Stearylbehenat, gehärtetem Rizinusöl, Palmitinsäure und Stearinsäure. Weitere besonders bevorzugte Lipid- oder Wachskomponenten-Mischungen a) sind ausgewählt aus Mischungen von C₂₀-C₄₀-Alkylstearat, gehärtetem Rizinusöl, Palmitinsäure und Stearinsäure.

Weitere bevorzugte erfindungsgemäße Deodorant- oder Antitranspirant-Stifte enthalten Lipid- oder Wachskomponente/n insgesamt in Mengen von 4 - 20 Gew.-%, bevorzugt 8 - 15 Gew.-%, bezogen auf die Gesamtzusammensetzung. In einer besonders bevorzugten Ausführungsform ist/sind der/die Ester aus einem gesättigten, einwertigen C₁₈-C₆₀-Alkohol und einer gesättigten C₈-C₃₆-Monocarbonsäure, der/die Lipid- oder Wachskomponente/n darstellt/ darstellen, in Mengen von insgesamt 2 - 10 Gew.-%, bevorzugt 2 - 6 Gew.-%, bezogen auf die Gesamtzusammensetzung, enthalten.

### ÖL-in-Wasser-Emulgatoren

Die erfindungsgemäßen Zusammensetzungen, die als Emulsion, insbesondere als Öl-in-Wasser-Emulsion oder Polyol-in-Wasser-Emulsion, formuliert sind, enthalten bevorzugt mindestens einen nichtionischen Öl-in-Wasser-Emulgator mit einem HLB-Wert von mehr als 7. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist.

Bei der Auswahl erfindungsgemäß geeigneter nichtionischer Öl-in-Wasser-Emulgatoren ist es besonders bevorzugt, ein Gemisch von nichtionischen Öl-in-Wasser-Emulgatoren einzusetzen, um die Stabilität der erfindungsgemäßen Stiftzusammensetzungen optimal einstellen zu können. Die einzelnen Emulgatorkomponenten liefern dabei einen Anteil zum Gesamt-HLB-Wert oder mittleren HLB-Wert des Öl-in-Wasser-Emulgatorgemisches gemäß ihrem Mengenanteil an der Gesamtmenge der Öl-in-Wasser-Emulgatoren. Erfindungsgemäß beträgt der mittlere HLB-Wert des Öl-in-Wasser- Emulgatorgemisches 10 - 19, bevorzugt 12 - 18 und besonders bevorzugt 14 - 17. Um derartige mittlere HLB-Werte zu erzielen, werden bevorzugt Öl-in-Wasser-Emulgatoren aus den HLB-Wertbereichen 10 - 14, 14 - 16 und gegebenenfalls 16 - 19 miteinander kombiniert. Selbstverständlich können die Öl-in-Wasser-Emulgatorgemische auch nichtionische Emulgatoren mit HLB-Werten im Bereich von > 7 - 10 und 19 - 20 enthalten; derartige Emulgatorgemische können erfindungsgemäß ebenfalls bevorzugt sein. Die erfindungsgemäßen Deodorant- oder Antitranspirant-Zusammensetzungen können aber in einer anderen bevorzugten Ausführungsform auch nur einen einzigen Öl-in-Wasser-Emulgator mit einem HLB-Wert im Bereich von 10 - 19 enthalten.

Bevorzugte erfindungsgemäße Deodorant- oder Antitranspirant-Zusammensetzungen sind dadurch gekennzeichnet, dass die nichtionischen Öl-in-Wasser-Emulgatoren ausgewählt sind aus ethoxylierten C₈-C₂₄-Alkanolen mit durchschnittlich 10 - 100 Mol Ethylenoxid pro Mol, ethoxylierten C₈-C₂₄-Carbonsäuren mit durchschnittlich 10 - 100 Mol Ethylenoxid pro Mol, Silicon-Copolyolen mit Ethylenoxid-Einheiten oder mit Ethylenoxid- und Propylenoxid-Einheiten, Alkylmono- und - oligoglycosiden mit 8 bis 22 Kohlenstoffatomen im Alkylrest und deren ethoxylierten Analoga, ethoxylierten Sterinen, Partialestern von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten C₈-C₂₄-Carbonsäuren haben die Formel R¹(OCH₂CH₂)ₙOH, wobei R¹ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-12, Ceteth-20, Ceteth-30, Steareth-12, Steareth-20, Steareth-30, Laureth-12 und Beheneth-20.

Weiterhin werden vorzugsweise C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 - 2, insbesondere bei 1,1 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet. Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, -Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexa glycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Besonders bevorzugte erfindungsgemäße Deodorant- oder Antitranspirant-Zusammensetzungen sind dadurch gekennzeichnet, dass der nichtionische Öl-in-Wasser-Emulgator in einer Gesamtmenge von 0,5 - 10 Gew.-%, besonders bevorzugt 1 - 4 Gew.-% und außerordentlich bevorzugt 1,5- 3 Gew.-%, bezogen auf die Gesamtzusammensetzung, enthalten ist.

### Wasser-in-Öl-Emulgatoren

Erfindungsgemäß bevorzugte Zusammensetzungen, die als Emulsion oder Stift formuliert sind, enthalten bevorzugt weiterhin mindestens einen nichtionischen Wasser-in-Öl-Emulgator mit einem HLB-Wert größer 1,0 und kleiner/gleich 7,0, ausgewählt aus den Mono- und Diestern von Ethylenglycol und den Mono-, Di-, Tri- und Tetraestern von Pentaerythrit mit linearen gesättigten Fettsäuren mit 12 - 30, insbesondere 14 - 22 Kohlenstoffatomen, die hydroxyliert sein können, sowie Mischungen hiervon, als Konsistenzgeber und/oder Wasserbinder. Erfindungsgemäß bevorzugt sind die Mono- und Diester. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß besonders bevorzugte nichtionische Wasser-in-Öl-Emulgatoren mit einem HLB-Wert größer 1,0 und kleiner/gleich 7,0 sind ausgewählt aus Pentaerythritylmonostearat, Pentaerythrityldistearat, Pentaerythrityltristearat, Pentaerythrityltetrastearat, Ethylenglycolmonostearat, Ethylenglycoldistearat sowie Mischungen hiervon. Erfindungsgemäß besonders bevorzugte Wasser-in-Öl-Emulgatoren mit einem HLB-Wert größer 1,0 und kleiner/ gleich 7,0 sind zum Beispiel als Handelsprodukte Cutina^{®} PES (INCI: Pentaerythrityl distearate), Cutina^{®} AGS (INCI: Glycol distearate) oder Cutina^{®} EGMS (INCI: Glycol stearate) erhältlich. Diese Handelsprodukte stellen bereits Mischungen aus Mono- und Diestern (bei den Pentaerythritylestern sind auch Tri- und Tetraester enthalten) dar. Erfindungsgemäß kann es bevorzugt sein, nur einen einzigen Wasser-in-Öl-Emulgator einzusetzen. In einer anderen bevorzugten Ausführungsform enthalten die erfindungsgemäßen Zusammensetzungen Mischungen, insbesondere technische Mischungen, von mindestens zwei Wasser-in-Öl-Emulgatoren. Unter einer technischen Mischung wird beispielsweise ein Handelsprodukt wie Cutina^{®} PES verstanden. Außer den genannten Wasser-in-Öl-Emulgatoren auf Basis der Ethylenglycol- oder Pentaerythritylester kann in einer bevorzugten Ausführungsform auch noch mindestens ein weiterer nichtionischer Wasser-in-Öl-Emulgator mit einem HLB-Wert- größer 1,0 und kleiner/gleich 7,0 enthalten sein, dessen Anteil an dem Gesamtgewicht an nichtionischen Wasser-in-Öl-Emulgatoren mit einem HLB-Wert größer 1,0 und kleiner/gleich 7,0 bevorzugt allerdings nicht größer als 80 % sein sollte. In einer besonders bevorzugten Ausführungsform enthalten die erfindungsgemäßen Zusammensetzungen den mindestens einen zusätzlichen Wasser-in-Öl-Emulgator mit einem HLB-Wert größer 1,0 und kleiner/ gleich 7,0 nur in einem Gewichtsanteil von maximal 10 % beziehungsweise sind frei von zusätzlichen Wasser-in-Öl-Emulgatoren. Einige dieser zusätzlichen geeigneten Emulgatoren sind beispielsweise in Kirk-Othmer, "Encyclopedia of Chemical Technology", 3. Aufl., 1979, Band 8, Seite 913, aufgelistet. Für ethoxylierte Addukte lässt sich der HLB-Wert, wie bereits erwähnt, auch berechnen.

Als Wasser-in-Öl-Emulgator bevorzugt geeignet sind:
- lineare gesättigte Alkanole mit 12 - 30 Kohlenstoffatomen, insbesondere mit 16 - 22 Kohlenstoffatomen, insbesondere Cetylalkohol, Stearylalkohol, Arachidylalkohol, Behenylalkohol und Lanolinalkohol oder Gemische dieser Alkohole, wie sie bei der technischen Hydrierung von pflanzlichen und tierischen Fettsäuren erhältlich sind,
- Ester und insbesondere Partialester aus einem Polyol mit 3 - 6 C-Atomen und linearen gesättigten und ungesättigten Fettsäuren mit 12 - 30, insbesondere 14 - 22 C-Atomen, die hydroxyliert sein können. Solche Ester oder Partialester sind z. B. die Mono- und Diester von Glycerin oder die Monoester von Propylenglycol mit linearen gesättigten und ungesättigten C₁₂
- C₃₀-Carbonsäuren, die hydroxyliert sein können, insbesondere diejenigen mit Palmitin- und Stearinsäure, die Sorbitanmono-, -di- oder -triester von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren und die Methylglucosemono- und -diester von linearen gesättigten und ungesättigten C₁₂ - C₃₀-Carbonsäuren, die hydroxyliert sein können;
- Sterine, also Steroide, die am C3-Atom des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z. B. Ergosterin, Stigmasterin, Sitosterin) und aus Pilzen und Hefen (Mykosterine) isoliert werden und die niedrig ethoxyliert (1 - 5 EO) sein können;
- Alkanole und Carbonsäuren mit jeweils 8 - 24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/gleich 7,0 aufweisen,
- Glycerinmonoether gesättigter und/oder ungesättigter, verzweigter und/oder unverzweigter Alkohole einer Kettenlänge von 8 - 30, insbesondere 12 - 18 Kohlenstoffatomen.
- Partialester von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert von kleiner/gleich 7 aufweisen,
- sowie Mischungen der vorgenannten Substanzen.

Erfindungsgemäß kann es bevorzugt sein, nur einen einzigen zusätzlichen Wasser-in-Öl-Emulgator einzusetzen. In einer anderen bevorzugter. Ausführungsform enthalten die erfindungsgemäßen Zusammensetzungen Mischungen, insbesondere technische Mischungen, von mindestens zwei zusätzlichen Wasser-in-Öl-Emulgatoren. Unter einer technischen Mischung wird beispielsweise ein Handelsprodukt wie Cutina^{®} GMS verstanden, das eine Mischung aus Glycerylmonostearat und Glyceryldistearat darstellt.

Besonders vorteilhaft einsetzbare zusätzliche Wasser-in-Öl-Emulgatoren sind Stearylalkohol, Cetylalkohol, Glycerylmonostearat, insbesondere in Form der Handelsprodukte Cutina^{®} GMS und Cutina^{®} MD (ex Cognis), Glyceryldistearat, Glycerylmonocaprinat, Glycerylmonocaprylat, Glycerylmonolaurat, Glycerylmonomyristat, Glycerylmonopalmitat, Glycerylmonohydroxystearat, Glycerylmonooleat, Glycerylmonolanolat, Glyceryldimyristat, Glyceryldipalmitat, Glyceryldioleat, Propylenglycolmonostearat, Propylenglycolmonolaurat, Sorbitanmonocaprylat, Sorbitanmonolaurat, Sorbitanmonomyristat, Sorbitanmonopalmitat, Sorbitanmonostearat, Sorbitansesquistearat, Sorbitandistearat, Sorbitandioleat, Sorbitansesquioleat, Saccharosedistearat, Arachidylalkohol, Behenylalkohol, Polyethylenglycol(2)stearylether (Steareth-2), Steareth-5, Oleth-2, Diglycerinmonostearat, Diglycerinmonoisostearat, Diglycerinmonooleat, Diglycerindihydroxystearat, Diglycerindistearat, Diglycerindioleat, Triglycerindistearat, Tetraglycerinmonostearat, Tetraglycerindistearat, Tetraglycerintristearat, Decaglycerinpentastearat, Decaglycerinpentahydroxystearat, Decaglycerinpentaisostearat, Decaglycerinpentaoleat, Soy Sterol, PEG-1 Soy Sterol, PEG-5 Soy Sterol, PEG-2-monolaurat und PEG-2-monostearat.

Besonders bevorzugte erfindungsgemäße Deodorant- oder Antitranspirant-Zusammensetzungen sind dadurch gekennzeichnet, dass mindestens ein Wasser-in-Öl-Emulgator in einer Gesamtmenge von 0,1 - 15 Gew.-%, bevorzugt 0,5 - 8,0 Gew.-%, und besonders bevorzugt 1 - 4 Gew.-%, bezogen auf die Gesamtzusammensetzung, enthalten sind. Weiterhin können auch Mengen von 2 - 3 Gew.-%, bezogen auf das Gesamtgewicht der Zusammensetzung, erfindungsgemäß außerordentlich bevorzugt sein.

In der nachfolgenden Tabelle sind verschiedene Öl-in-Wasser-Emulgatoren und Wasser-in-Öl-Emulgatoren und ihre HLB-Werte zusammengestellt. Die HLB-Werte können aber auch nach Griffin berechnet werden, wie es beispielsweise im RÖMPP Chemie Lexikon, insbesondere in der Online-Version von November 2003, und den dort unter dem Stichwort "HLB-System" zitierten Handbüchern von Fiedler, Kirk-Othmer und Janistyn dargestellt beziehungsweise tabelliert ist. Sofern es in der Literatur unterschiedliche Angaben zum HLB-Wert einer Substanz gibt, sollte derjenige HLB-Wert für die erfindungsgemäße Lehre genutzt werden, der dem nach Griffin berechneten Wert am nächsten kommt. Falls sich auf diese Art und Weise kein eindeutiger HLB-Wert ermitteln lässt, ist der HLB-Wert, den der Hersteller des Emulgators angibt, für die erfindungsgemäße Lehre zu nutzen. Falls auch dies nicht möglich ist, ist der HLB-Wert experimentell zu ermitteln.

HLB-Wert Chemische Bezeichnung (aus H. Janistyn, Handbuch der Kosmetika und Riechstoffe, Hüthig-Verlag Heidelberg, 3. Auflage, 1978, Band 1, Seite 470 und Band 3, Seiten 68 - 78))

| | |
|---|---|
| 1 | Triglyceride gesättigter Fettsäuren |
| | Glyceryltrioleat |
| 1,5 | Ethylenglycoldistearat |
| 1,6 | Pur-Cellinöl |
| 1,8 | Sorbitantrioleat |
| | Glycerindioleat |
| 2,1 | Sorbitantristearat |
| 2,4 | Propylenglycollactostearat |
| 2,7 | Glycerinmonooleat |
| | Sorbitdioleat |
| 2,8 | Glycerinmonostearat |
| | Propylenglycolmono-/distearat, nicht selbstemulgierend |
| 2,9 | Ethylenglycolmonostearat |
| 3,0 | Decaglycerindecaoleat |
| | Decaglycerindecastearat |
| | Generol 122 (Rapeseed Sterols) |
| | Sucrosedistearat |
| 3,1 | Decaglycerindecaoleat |
| | Glycerylmononcinoleat |
| | Pentaerythnitylmonostearat |
| | Pentaerythrilylsesquioleat |
| 3,2 | Ethylenglycolmonodistearat, nicht sebstemulgierend |
| | Glycolstearat |
| 3,3 | Glycerinmonolaurat |
| 3,4 | Propylenglycolmonostearat |
| 3,5 | Ethylenglycolmonostearat |
| | Pentaerythritylmonooleat |
| | Polyethylenglycol(100)monooleat |
| 3,6 | Glycerinmono-/dioleat, nicht selbstemulgierend |
| | Monoethoxylaurylether |
| 3,7 | Sorbitansesquioleat (Dehymuls SSO) |
| 3,8 | Glycerinmonodistearat, nicht selbstemulgierend |
| | Polyethylenglycol(100)monostearat |
| | Diglycerinsesquioleat |
| | N,N-Dimethylcaproamid |
| | Pentaerythritmonotallat |
| | Propylenglycolmonolaurat |
| 4,0 | Decaglycerinoctaoleat |
| 4,3 | Sorbitanmonooleat (Dehymuls SMO) |
| | Diethylenglycolmonostearat |
| 4,4 | 1.2-Propylenglycolmonodistearat, selbstemulgierend |
| 4,5 | Glycerinmonostearatpalmitat (90 %), nicht selbstemulgierend |
| | Propylenglycolmonolaurat |
| 4,7 | Sorbitanmonostearat (Dehymuls SMS) |
| | Diethylenglycolmonooleat |
| 4,8 | Pentaerythritmonolaurat |
| 4,9 | Polyoxyethylen(2)oleylalkohol (Polyoxyethylen(2)oleylether) |
| | Polyoxyethylen(2)stearylalkohol (Polyoxyethylen(2)stearylether) |
| 5,0 | Ethylenglycolmonodistearat |
| | Generol 122 E 5 (PEG-5 Soy Sterol) |
| | Polyethylenglycol(100)monoricinoleat |
| | Polyethylenglycol(200)distearat |
| | Polyglyceryl-3-isostearate (z. B. Isolan Gl 34 von Tego) |
| 5,9 | Polyethylenglycol(200)dilaurat |
| 6,0 | Decaglycerintetraoleat |
| | Polyethylenglycol(100)monolaurat |
| | Polyethylenglycol(200)dioleat |
| 6,1 | Diethylenglycolmonolaurat (Diglycollaurat) |
| 6,3 | Polyethylenglycol(300)dilaurat |
| 6,4 | Glycerinmonoricinoleat |
| | Glycerinsorbitanmonolaurat |
| 6,5 | Diethylenglycolmonolaurat |
| | Natriumstearoyl-2-lactylat |
| 6,7 | Sorbitanmonopalmitat |
| 6,8 | Glycerinmonococoat |
| | Glycerinmonolaurat |
| 7,0 | Polyoxyethylen(2)C₁₀-C₁₄-fettalkoholether, Laureth-2 (Dehydol LS 2) |
| | Saccharosedistearat |
| 7,2 | Polyethylenglycol(400)dioleat |
| | Saccharosedioleat |
| 7,4 | Polyethylenglycol(100)monolaurat |
| | Saccharosedipalmitat |
| 7,5 | Saccharosedipalmitat |
| 7,6 | Glycerinsorbitanlaurat |
| 7,8 | Polyethylenglycol(400)distearat |
| 7,9 | Polyethylenglycol(200)monostearat |
| | Polyoxyethylen(3)tridecylalkohol |
| 8-8,2 | Polyethylenglycol(400)distearat |
| 8,0 | Polyoxyethylen(3)C₁₀-C₁₄-fettalkoholether, Laureth-3 (Dehydol LS 3) |
| | N.N-Dimethyllauramid |
| | Natriumlauroyllactylat, Natriumlauroyl-2-lactylat |
| | Polyethylenglycol(200)monooleat |
| | Polyethylenglycol(220)monotallat |
| | Polyethylenglycol(1500)dioleat |
| | Polyoxyethylen(4)oleylalkohol |
| | Polyoxyethylen(4)stearylcetylether |
| 8,2 | Triglycerinmonooleat |
| 8,3 | Diethylenglycolmonolaurat |
| 8,4 | Polyoxyethylen(4)cetylether |
| | Polyoxyethylenglycol(400)dioleat |
| 8,5 | Natriumcaproyllactylat |
| | Polyethylenglycol(200)monostearat |
| | Sorbitanmonooleat |
| 8,6 | Sorbitanmonolaurat (Dehymuls SML) |
| | Polyethylenglycol(200)monolaurat |
| 8,8 | Polyoxyethylen(4)myristylether |
| | Polyethylenglycol (400)dioleat |
| 8,9 | Nonylphenol, polyoxyethyliert mit 4 Mol EO |
| 9,0 | Oleth-5 (z. B. Eumulgin O 5) |
| 9,2-9,7 | Polyoxyethylen(4)laurylalkohol (je nach Handelsprodukt, z. B. Brij 30, Dehydol LS 4) |
| 9,3 | Polyoxyethylen(4)tridecylalkohol |
| 9,6 | Polyoxyethylen(4)sorbitanmonosteatat |
| 9,8 | Polyethylenglycol(200)monolaurat |
| 10-11 | Polylethylenglycol(400)monooleat |
| 10,0 | Didodecyldimethylammoniumchlorid |
| 10,0 | Polyethylenglycol(200)monolaurat |
| | Polyethylenglycol(400)dilaurat |
| | Polyethylenglycol(600)dioleat |
| | Polyoxyethylen(4)sorbitanmonostearat |
| | Polyoxyethylen(5)sorbitanmonooleat |
| 10,2 | Polyoxyethylen(40)sorbitol hexaoleat |
| 10,4-10,6 | Polyoxyethylenglycol(600)distearat |
| 10,5 | Polyoxyethylen(20)sorbitantristearat |
| 10,6 | Saccharosemonostearat |
| 10,7 | Saccharosemonooleat |
| 11 - 11,4 | Polyethylenglycol(400)monooleat |
| 11,0 | Polyethylenglycol(350)monostearat |
| | Polyethylenglycol(400)monotallat |
| | Polyoxyethylenglycol(7)monostearat |
| | Polyoxyethylenglycol(8)monooleat |
| | Polyoxyethylen(20)sorbitantrioleat |
| | Polyoxyethylen(6)tridecylalkohol |
| 11,1 | Polyethylenglycol(400)monostearat |
| 11,2 | Polyoxyethylen(9)monostearat |
| | Saccharosemonooleat |
| | Saccharosemonostearat |
| 11,4 | Polyoxyethylen(50)sorbitol hexaoleat |
| | Saccharosemonotallat |
| | Saccharosestearatpalmitat |
| 11,6 | Polyoxyethylenglycol(400)monoricinoleat |
| 11,7 | Saccharosemonomyristat |
| | Sacccharosemonopalmitat |
| 12,0 | PEG-10 Soy Sterol (z. B. Generol 122 E 10) |
| | Triethanolaminoleat |
| 12,2-12,3 | Nonylphenol, ethoxyliert mit 8 Mol EO |
| 12,2 | Saccharosemonomyristat |
| 12,4 | Saccharosemonolaurat |
| | Polyoxyethylen(10)oleylalkohol, Polyoxyethylen(10)oleylether |
| | Polyoxyethylen(10)stearylalkohol, Polyoxyethylen(10)stearylether |
| 12,5 | Polyoxymethylen(10)stearylcetylether |
| 12,7 | Polyoxyethylen(8)tridecylalkohol |
| 12,8 | Poyoxyethylengycol(400)monolaurat |
| | Saccharosemonococoat |
| 12,9 | Polyoxyethylen(10)cetylether |
| 13 | Glycerinmonostearat, ethoxyliert (20 Mol EO) |
| 13,0 | Eumulgin O 10 |
| | Eumulgin 286 |
| | Eumulgin B 1 (Ceteareth-12) |
| 13,0 | C12-Fettamine, ethoxyliert (5 Mol EO) |
| 13,1 | Nonylphenol, ethoxyliert (9,5 Mol EO) |
| 13,2 | Polyethylengycol(600)monostearat |
| | Polyoxyethylen(16)tallöl |
| 13,3 | Polyoxyethylen(4)sorbitanmonolaurat |
| 13,5 | Nonylphenol, ethoxyliert (10,5 Mol EO) |
| | Polyethylenglycol(600)monooleat |
| 13,7 | Polyoxyethylen(10)tridecylalkohol |
| | Polyethylenglycol(660)monotallat |
| | Polyethylenglycol(1500)monostearat |
| | Polyoxyethylenglycol(1500)dioleat |
| 13,9 | Polyethylenglycol(400)monococoat |
| | Polyoxyethylen(9)monolaurat |
| 14-16 | Eumulgin HRE 40 (Ricinusöl, mit 40 EO ethoxyliert und hydriert) |
| 14,0 | Polyoxyethylen(12)laurylether |
| | Polyoxyethylen(12)tridecylalkohol |
| 14,2 | Polyoxyethylen(15)stearylalkohol |
| 14,3 | Polyoxyethylen(15)stearylcetylether |
| 14,4 | Gemisch aus C12-C15-Fettalkoholen mit 12 Mol EO |
| 14,5 | Polyoxyethylen(12)laurylalkohol |
| 14,8 | Polyoxyethylenglycol(600)monolaurat |
| 14,9 - 15,2 | Sorbitanmonostearat, mit 20 EO ethoxyliert (z. B. Eumulgin SMS 20) |
| 15 - 15,9 | Sorbitanmonooleat, mit 20 EO ethoxyliert (z. B. Eumulgin SMO 20) |
| 15,0 | PEG-20 Glyceryl stearate (z. B. Cutina E 24) |
| | PEG-40 Castor Oil (z. B. Eumulgin RO 40) |
| | Decylglucosid (Oramix NS 10) |
| | Dodecylglucosid (Plantaren APG 600) |
| | Dodecyltrimethylammoniumchlorid |
| | Nonylphenol, ethoxyliert mit 15 Mol EO |
| | Polyethylenglycol(1000)monostearat |
| | Polyoxyethylen(600)monooleat |
| 15-17 | Eumulgin HRE 60 (Ricinusöl, mit 60 EO ethoxyliert und hydriert) |
| 15,3 | C12-Fettamine, polyoxyethyliert mit 12 Mol EO |
| | Polyoxyethylen(20)oleylalkohol, Polyoxyethylen(20)oleylether |
| 15,4 | Polyoxyethylen(20)stearylcetylether (z. B. Eumulgin B 2 (Ceteareth-20)) |
| 15,5 | Polyoxyethylen(20)stearylalkohol |
| 15,6 | Polyoxyethylenglycol(1000)monostearat |
| | Polyoxyethylen(20)sorbitanmonopalmitat |
| 15,7 | Polyoxyethylen(20)cetylether |
| 15,9 | Dinatriumtriethanolamindistearylheptaglycolethersulfosuccinat |
| 16,0 | Nonylphenol ethoxyliert mit 20 Mol EO |
| | Polyoxyethylen(25)propylenglycolstearat |
| 16 - 16,8 | Polyoxyethylen(30)monostearat |
| 16,3-16,9 | Polyoxyethylen(40)monostearat |
| 16,5 - 16,7 | Polyoxyethylen(20)sorbitanmonolaurat (z. B. Eumulgin SML 20) |
| 16,6 | Polyoxyethylen(20)sorbit |
| 16,7 | C18-Fettamine, polyoxyethyliert mit 5 Mol EO |
| | Polyoxyethylen(23)laurylalkohol |
| 17,0 | Ceteareth-30, z. B. Eumulgin B 3 |
| | Octylglucosid (Triton CG 110) |
| | Polyoxyethylen(30)glycerylmonolaurat |
| 17,1 | Nonylphenol, ethoxyliert mit 30 Mol EO |
| 17,4 | Polyoxyethylen(40)stearylalkohol |

Weitere bevorzugte erfindungsgemäße Zusammensetzungen sind dadurch gekennzeichnet, dass der Gesamtgehalt an nichtionischen und ionischen Emulgatoren und/oder Tensiden mit einem HLB-Wert über 8 maximal 20 Gew.-%, bevorzugt maximal 15 Gew.-%, besonders bevorzugt maximal 10 Gew.-%, besonders bevorzugt maximal 7 Gew.-%, weiterhin besonders bevorzugt maximal 4 Gew.-% und außerordentlich bevorzugt maximal 3 Gew.-%, jeweils bezogen auf die gesamte erfindungsgemäße Zusammensetzung, beträgt.

Bevorzugte erfindungsgemäße Zusammensetzungen, die als Emulsion, Suspension oder Stift vorliegen, enthalten bevorzugt weiterhin mindestens ein bei 20 ° C flüssiges Öl, das keine Duftstoffkomponente und kein etherisches Öl darstellt. Erfindungsgemäß bevorzugte Öle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol, Octyldodecanol und 2-Ethylhexylalkohol. Weitere bevorzugte Ölkomponenten sind Mischungen aus Guerbetalkoholen und Guerbetalkoholestern, z.B. das Handelsprodukt Cetiol^{®} PGL (Hexyldecanol und Hexyldecyllaurat).

Weitere erfindungsgemäß bevorzugte Öle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltrilsostearin und die Handelsprodukte Estol^{®} GTEH 3609 (Uniqema) oder Myritol^{®} GTEH (Cognis) mit verzweigten Fettsäureresten.

Weitere erfindungsgemäß besonders bevorzugte Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte Öle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).

Weitere erfindungsgemäß bevorzugte Ölkomponenten sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Hexyldecylstearat (Eutanol^{®} G 16 S), Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bedingt geeignet sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat.

Weitere erfindungsgemäß bevorzugte Ölkomponenten sind ausgewählt aus den Anlagerungsprodukten von mindestens 6 Ethylenoxid und/oder Propylenoxid-Einheiten an ein- oder mehrwertige C₃₋₂₂-Alkanole wie Butanol, Butandiol, Myristylalkohol und Stearylalkohol, z. B. PPG-14-Butylether (Ucon Fluid^{®} AP), PPG-9-Butylether (Breox^{®} B25), PPG-10-Butandiol (Macor^{®} 57) und PPG-15-Stearylether (Arlamol^{®} E).

Weitere efindungsgemäß bevorzugte Ölkomponenten sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI.

Weitere erfindungsgemäß bevorzugte Ölkomponenten sind ausgewählt aus den symmetrischen, unsymmetrischen oder cyclischen Estern der Kohlensäure mit Fettalkoholen, z. B. Glycerincarbonat, Dicaprylylcarbonat (Cetiol^{®} CC) oder die Ester der DE 197 56 454 A1.

Weitere erfindungsgemäß bevorzugte Ölkomponenten sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Es kann erfindungsgemäß außerordentlich bevorzugt sein, Mischungen der vorgenannten Öle einzusetzen. Weitere erfindungsgemäß bevorzugte Ölkomponenten sind ausgewählt aus Siliconölen und Kohlenwasserstoffölen.

Erfindungsgemäß bevorzugte Siliconöle sind ausgewählt aus Dialkyl- und Alkylarylsiloxanen, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen.

Weitere erfindungsgemäß bevorzugte Siliconöle sind ausgewählt aus flüchtigen Siliconölen, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind, oder linear, z. B. Hexamethyldisiloxan (L₂), Octamethyltrisiloxan (L₃), Decamethyltetrasiloxan (L₄), beliebige Zweier- und Dreiermischungen aus L₂, L₃ und/oder L₄, wie sie z. B. in den Handelsprodukten DC 2-1184, Dow Coming^{®} 200 (0, 65 cSt) und Dow Corning^{®} 200 (1,5 cSt) von Dow Corning enthalten sind.

Weitere erfindungsgemäß bevorzugte Siliconöle sind ausgewählt aus nichtflüchtigen höhermolekularen linearen Dimethylpolysiloxanen, im Handel erhältlich z. B. unter der Bezeichnung Dow Corning^{®} 190, Dow Corning^{®} 200 Fluid mit Viskositäten im Bereich von 5 - 100 cSt, bevorzugt 5 - 50 cSt oder auch 5 - 10 cSt, und Baysilon^{®} 350 M.

Erfindungsgemäß bevorzugte natürliche und synthetische Kohlenwasserstoffe sind ausgewählt aus Paraffinölen, Isohexadecan, Isoeicosan, Polyisobutenen und Polydecenen, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan. Besonders bevorzugte erfindungsgemäße Deodorant- oder Antitranspirant-Zusammensetzungen sind dadurch gekennzeichnet, dass das/die bei 20 °C flüssige/n Öl/e in einer Gesamtmenge von 0,1 - 80 Gew.-%, bevorzugt 2 - 20 Gew.-%, besonders bevorzugt 3 - 15 Gew.-%, jeweils bezogen auf das Gesamtgewicht der Zusammensetzung, enthalten ist/sind.

In einer weiteren bevorzugten Ausführungsform der Erfindung weist ein Anteil der Ölkomponenten von mindestens 80 Gew.-% einen Brechungsindex n_{D} von 1,39 -1,51 auf. Besonders bevorzugt ist es, wenn 5 - 40 - 50 Gew.-%, außerordentlich bevorzugt 10 - 12 - 25 - 30 Gew.-% der Ölkomponenten einen Brechungsindex n_{D} von 1,43 - 1,51, bevorzugt 1,44 - 1,49, besonders bevorzugt 1,45 - 1,47 - 1,485, bei 20 °C (gemessen bei λ = 589 nm) aufweisen.

Erfindungsgemäß besonders bevorzugte Zusammensetzungen, die als Wasser-in-Öl-Emulsion konfektioniert sind, enthalten bevorzugt weiterhin mindestens einen Wasser-in-Öl-Emulgator. Der mindestens eine Wasser-in-Öl-Emulgator ist bevorzugt in einer Menge von 0,5 - 5 Gew.-%, besonders bevorzugt 1,0 - 1,5 - 2,5 Gew.-%, jeweils bezogen auf das Gesamtgewicht der Emulsion, enthalten.

Eine erfindungsgemäß besonders bevorzugte Gruppe von Wasser-in-Öl-Emulgatoren sind die Poly-(C₂-C₃)alkylenglycol-modifizierten Silicone, deren frühere INCI-Bezeichnung Dimethicone Copolyol lautete, mit den aktuellen INCI-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 20, insbesondere 14 - 18, stehen), Bis-PEG/PPG-c/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14 - 18 und besonders bevorzugt 16, stehen). Besonders bevorzugt sind PEG/PPG-18/18 Dimethicone, das in einer 1:9-Mischung mit Cyclomethicone als DC 3225 C bzw. DC 5225 C im Handel erhältlich ist, PEG/PPG-4/12 Dimethicone, das unter der Bezeichnung Abil B 8852 erhältlich ist, sowie Bis--PEG/PPG-14/14-Dimethicone; das in einer Mischung mit Cyclomethicone als Abil EM 97 (Goldschmidt) im Handel erhältlich ist, Bis-PEG/PPG-20/20 Dimethicone, das unter der Bezeichnung Abil B 8832 erhältlich ist, PEG/PPG-5/3 Trisiloxane (Silsoft 305), sowie PEG/PPG-20/23 Dimethicone (Silsoft 430 und Silsoft 440).

Weitere erfindungsgemäß bevorzugte W/O-Emulgatoren sind Poly-(C₂-C₃)alkylenglycol-modifizierte Silicone, die mit C₄-C₁₈-Alkylgruppen hydrophob modifiziert sind, besonders bevorzugt Cetyl PEG/PPG-10/1 Dimethicone (früher: Cetyl Dimethicone Copolyol, erhältlich als Abil EM 90 oder in einer Mischung aus Polyglyceryl-4-isostearat, Cetyl PEG/PPG-10/1 Dimethicone und Hexyllaurat unter der Handelsbezeichnung Abil WE 09), weiterhin Alkyl Methicone Copolyole und Alkyl Dimethicone Ethoxy Glucoside.

Erfindungsgemäß besonders bevorzugte Zusammensetzungen enthalten weiterhin bevorzugt mindestens einen hautkühlenden Wirkstoff enthalten. Erfindungsgemäß geeignete hautkühlende Wirkstoffe sind beispielsweise Menthol, Isopulegol sowie Mentholderivate, z. B. Menthyllactat, Menthylglycolat, Menthylpyrrolidoncarbonsäure, Menthylmethylether, Menthoxypropandiol, Menthonglycerinacetal (9-Methyl-6-(1-methylethyl)-1,4- dioxaspiro (4.5)decan-2-methanol), Monomenthylsuccinat und 2-Hydroxymethyl-3,5,5-trimethylcyclohexanol. Als hautkühlende Wirkstoffe bevorzugt sind Menthol, Isopulegol, Menthyllactat, Menthoxypropandiol und Menthylpyrrolidoncarbonsäure sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Erfindungsgemäß besonders bevorzugt ist, dass mindestens ein hautkühlender Wirkstoff in einer Gesamtmenge von 0,01 - 1 Gew.%, besonders bevorzugt 0,02 - 0,5 Gew.% und außerordentlich bevorzugt 0,05 - 0,2 Gew.%, jeweils bezogen auf das Gesamtgewicht der Zusammensetzung, enthalten ist.

Erfindungsgemäß besonders bevorzugte Zusammensetzungen, die als treibgasgetriebenes Aerosol konfektioniert sind, enthalten mindestens ein Treibmittel. Bevorzugte Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann.

Die Menge der Treibmittel beträgt bevorzugt 20 - 80 Gew.-%, besonders bevorzugt 30 - 70 Gew.% und außerordentlich bevorzugt 40 - 50 Gew.-%, jeweils bezogen auf das Gesamtgewicht der Zubereitung, bestehend aus der erfindungsgemäßen Zusammensetzung und dem Treibmittel.

Als Druckgasbehälter kommen Gefäße aus Metall (Aluminium, Weißblech, Zinn), geschütztem bzw. nicht-splitterndem. Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der erfindungsgemäßen Zusammensetzung.

Bevorzugte erfindungsgemäße Zusammensetzungen enthalten weiterhin mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Bevorzugte erfindungsgemäße Deodorant- oder Antitranspirant-Zusammensetzungen sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-octandiol, 1,8-Octandiol, Dipropylenglycol, Tripropylenglycol, Diglycerin, Triglycerin, Erythrit, Sorbit sowie Mischungen der vorgenannten Substanzen.

Besonders bevorzugte erfindungsgemäße Deodorant- oder Antitranspirant-Zusammensetzungen sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten insgesamt in Mengen von 3 - 30 Gew.-%, bevorzugt 8 - 25 Gew.-%, besonders bevorzugt 10 - 18 Gew.-%, jeweils bezogen auf die Gesamtzusammensetzung, enthalten ist.

Bevorzugte erfindungsgemäße Zusammensetzungen enthalten Wasser. Der Anteil des Wassers beträgt bevorzugt 0,1 - 99 Gew.-%, besonders bevorzugt 3 bis 80 Gew.-%, außerordentlich bevorzugt 10 bis 70 Gew.-%, weiterhin bevorzugt 15 - 60 Gew.-%, 20 - 50 Gew.-%, 30 - 40 Gew.-%, jeweils bezogen auf die Gesamtzusammensetzung.

Besonders bevorzugte erfindungsgemäße Deodorant- oder Antitranspirant-Zusammensetzungen sind dadurch gekennzeichnet, dass mindestens eine Lipid- oder Wachskomponente mit einem Schmelzpunkt im Bereich von 25 - < 50°C, ausgewählt aus Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen, enthalten ist. Diese niedriger schmelzenden Lipid- oder Wachskomponenten ermöglichen eine Konsistenzoptimierung stiftförmiger oder cremeförmiger Produkte und eine Minimierung der sichtbaren Rückstände auf der Haut. Besonders bevorzugt sind Handelsprodukte mit der INCI-Bezeichnung Cocoglycerides, insbesondere die Handelsprodukte Novata^{®} (ex Cognis), besonders bevorzugt Novata^{®} AB, ein Gemisch aus C₁₂-C₁₈-Mono-, Di- und Triglyceriden, das im Bereich von 30 - 32°C schmilzt, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂-C₁₈-Alkoholen mit gesättigten C₁₂-C₁₈-Monocarbonsäuren sind Stearyllaurat, Cetearylstearat (z. B. Crodamol^{®} CSS), Cetylpalmitat (z. B. Cutina^{®} CP) und Myristylmyristat (z. B. Cetiol^{®} MM).

Weitere besonders bevorzugte erfindungsgemäße Deodorant- oder Antitranspirant-Zusammensetzungen sind dadurch gekennzeichnet, dass die mindestens eine Lipid- oder Wachskomponente mit einem Schmelzpunkt im Bereich von 25 - < 50°C in Mengen von 0,01 bis 20 Gew.-%, bevorzugt 3 - 20 Gew.-%, besonders bevorzugt 5 - 18 Gew.-% und außerordentlich bevorzugt 6 - 15 Gew.-%, bezogen auf die Gesamtzusammensetzung, enthalten ist.

Besonders bevorzugte erfindungsgemäße Deodorant- oder Antitranspirant-Zusammensetzungen sind dadurch gekennzeichnet, dass sie zur Verbesserung der Stiftkonsistenz und der sensorischen Eigenschaften weiterhin mindestens einen festen, wasserunlöslichen teilchenförmigen Füllstoff enthalten. In einer außerordentlich bevorzugten Ausführungsform ist dieser Füllstoff ausgewählt aus gegebenenfalls modifizierten Stärken (z. B. aus Mais, Reis, Kartoffeln) und Stärkederivaten, die gewünschtenfalls vorverkleistert sind, insbesondere Stärkederivaten vom Typ DRY FLO^{®}, Cellulose und Cellulosederivaten, Siliciumdioxid, Kieselsäuren, z. B. Aerosil^{®}-Typen, sphärischen Polyalkylsesquisiloxan-Partikeln (insbesondere Aerosil^{®} R972 und Aerosil^{®} 200V von Degussa), Kieselgelen, Talkum, Kaolin, Tonen, z. B. Bentoniten, Magnesiumaluminiumsilikaten, Bornitrid, Lactoglobulinderivaten, z. B. Natrium-C₈₋₁₆-Isoalkylsuccinyllactoglobulinsulfonat, von Brooks Industries erhältlich als Handelsprodukt Biopol^{®} OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen. Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere Polymerpulver, die sich für den erfindungsgemäßen Zweck eignen, sind z. B. Polymethacrylate (Micropearl^{®} M von SEPPIC oder Plastic Powder A von NIKKOL), Styrol-Divinylbenzol-Copolymeren (Plastic Powder FP von NIKKOL), Polyethylen- und Polypropylen-Pulver (ACCUREL^{®} EP 400 von AKZO) oder auch Siliconpolymere (Silicone Powder X2-1605 von Dow Corning). Besonders bevorzugte erfindungsgemäße Deodorant- oder Antitranspirant-Zusammensetzungen enthalten mindestens einen festen, wasserunlöslichen teilchenförmigen Füllstoff in einer Gesamtmenge von 0,01 bis 30 Gew.-%, bevorzugt 5 - 20 Gew.-%, besonders bevorzugt 8 - 15 Gew.-%, jeweils bezogen auf die Gesamtzusammensetzung.

Besonders bevorzugte erfindungsgemäße Deodorant- oder Antitranspirant-Zusammensetzungen sind dadurch gekennzeichnet, dass weiterhin mindestens eine Duftstoffkomponente enthalten ist. Als Duftstoffkomponente können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyral, p-tert.-Butylcyclohexylacelat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellytoxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citroneliol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnole erzeugen.

Besonders bevorzugte erfindungsgemäße Deodorant- oder Antitranspirant-Zusammensetzungen sind dadurch gekennzeichnet, dass mindestens eine Duftstoffkomponente in einer Gesamtmenge von 0,00001 bis 4 Gew.-%, bevorzugt 0,5 - 2 Gew.-%, jeweils bezogen auf die Gesamtzusammensetzung, enthalten ist.

Weitere Gegenstände sind, wie weiter oben erwähnt, ein Verfahren zur Verringerung des Haarwuchses, wobei eine erfindungsgemäße Deodorant- oder Antitranspirant-Zusammensetzung topisch in einer wirksamen Menge auf die Haut, insbesondere auf die Haut im Achselbereich, aufgetragen wird und ein Verfahren zur Behandlung von Körpergeruch, bei dem eine erfindungsgemäße Zubereitung auf die Haut aufgetragen wird.

Bezüglich bevorzugter Ausführungsformen der erfindungsgemäßen Verfahren gilt mutatis mutandis das zu den erfindungsgemäßen Zusammensetzungen Gesagte.

### Ausführungsbeispiele

Die nachfolgenden Formulierungsbeispiele sollen den Gegenstand der Erfindung erläutern, ohne ihn hierauf zu beschränken.

Erfindungsgemäße Antitranspirant-Stifte auf Basis einer Öl-in-Wasser-Emulsion (Mengenangaben in Gew.-%)

| Beispiel Nr. | 1 | 2 | 3 | 4 | 5 | 6 | 7 |
|---|---|---|---|---|---|---|---|
| Komponente | | | | | | | |
| Cutina^{®} AGS | 2,5 | 2,5 | - | - | - | 2,5 | 2,5 |
| Cutina^{®} EGMS | - | - | 2,5 | 2 | - | - | - |
| Cutina^{®} PES | - | - | - | - | 2 | - | - |
| Cutina^{®} FS45 | 3,5 | 3,5 | 3,5 | 3,5 | 3,5 | 3,5 | 3,5 |
| Eumulgin^{®} B2 | 0,8 | 0,8 | 0,8 | 0,8 | 0,8 | 0,8 | 0,8 |
| Eumulgin^{®} B3 | 0,8 | 0,8 | 0,8 | 0,8 | 0,8 | 0,8 | 0,8 |
| Diisopropyladipat (Ceraphyl^{®} 230) | 6 | 6 | 6 | 6 | 6 | 6 | 6 |
| Novata^{®} AB | 4 | 4 | 4 | 4 | 4 | 4 | 4 |
| Cutina^{®} CP | 5 | 5 | 5 | 5 | 5 | 5 | 5 |
| Cutina^{®} HR | 4 | 4 | 4 | 4 | 4 | 4 | 4 |
| Kesterwachs K62 | 5 | 5 | 5 | 5 | 5 | 5 | 5 |
| Locron^{®} L (ACH-Lösung 50%ig) | 40 | 40 | 40 | 40 | 40 | 40 | 40 |
| Talkum Pharma G | 10 | 10 | 10 | 10 | 10 | 10 | 10 |
| Parfüm | 1,2 | 1,2 | 1,2 | 1,2 | 1,2 | 1,2 | 1,2 |
| Sensiva SC 50 | - | - | 0,6 | 0,6 | 0,6 | 0,6 | 0,6 |
| Tinogard Q | 0,025 | 0,05 | 0,025 | - | 0,1 | 0,1 | - |
| Tinogard AS | - | - | 0,025 | - | - | - | - |
| 1,2-Propandiol | 10 | 10 | 10 | 10 | 10 | 10 | 10 |
| Maltosin | 0,1 | 0,2 | 0,5 | 1,0 | 1,0 | 1,5 | 2,0 |
| Lactose | - | - | - | 0,1 | 0,01 | 0,1 | 0,05 |
| Glucose | - | - | - | 0,1 | - | - | - |
| Wasser, vollentsalzt | ad 100,0 | ad 100,0 | ad 100,0 | ad 100,0 | ad 100,0 | ad 100,0 | ad 100,0 |

### Beispiel 8: Erfindungsgemäße Antitranspirant-Emulsion (O/W)

| INGREDIENTS | Gew.-%-Anteil |
|---|---|
| ALUMINUM ZIRCONIUM TETRACHLOROHYDREX GLY | 23,7 |
| STEARETH-2 | 2,4 |
| STEARETH-21 | 1,6 |
| PARFUM | 1,2 |
| PPG-15 STEARYL ETHER | 0,5 |
| ALUMINUM STARCH OCTENYLSUCCINATE | 0,1 |
| TOCOPHERYL ACETATE | 0,5 |
| Maltosin | 0,5 |
| AQUA | ad 100,0 |

Die Emulsion gemäß Beispiel 8 wies am ersten Tag nach der Herstellung eine Viskosität von 2200 mPas auf, gemessen mit einem Brookfield-Viskosimeter, Spindel RV 4, 20 s-1, ohne Helipath, bei 20 °C Umgebungstemperatur und 20 °C Probentemperatur; sie wurde in einen Roll-on-Applikator abgefüllt.

### Beispiel 9: erfindungsgemäße Antitranspirant-Emulsion (O/W)

| INGREDIENTS | Gew.-%-Anteil |
|---|---|
| ALUMINUM CHLOROHYDRATE | 20,00 |
| STEARETH-2 | 2,30 |
| STEARETH-21 | 1,50 |
| PARFUM | 1,00 |
| PPG-15 STEARYL ETHER | 0,50 |
| ALUMINUM STARCH OCTENYLSUCCINATE | 0,10 |
| Maltosin | 0,75 |
| ISOPROPYL MYRISTATE | 0,10 |
| AQUA | ad 100,00 |

Die Emulsion gemäß Beispiel 9 wies am ersten Tag nach der Herstellung eine Viskosität von 1700 mPas auf, gemessen mit einem Brookfield-Viskosimeter, Spindel RV 4, 20 s-1, ohne Helipath, bei 20 °C Umgebungstemperatur und 20 °C Probentemperatur; sie wurde in einen Roll-on-Applikator abgefüllt.

Wasserfreie tensidhaltige Antitranspirant-Stifte (Angaben in Gewichtsteilen)

| | 10.1 | 10.2 | 10.3 | 10.4 | 10.5 | 10.6 | 10.7 | 10.8 | 10.9 |
|---|---|---|---|---|---|---|---|---|---|
| Siliconöl DC^{®} 245 | ad 100,0 | ad 100,0 | ad 100,0 | ad 100,0 | ad 100,0 | ad 100,0 | ad 100,0 | ad 100,0 | ad 100,0 |
| Eutanol^{®} G 16 | 10 | - | - | 15 | 10 | - | 10 | - | 10 |
| Cetiol^{®} OE | - | 10 | 15 | - | - | - | - | - | - |
| Ucon Fluid^{®} AP | 5 | 5 | 5 | 5 | 5 | 5 | 5 | 5 | 5 |
| Cutina^{®} HR | 6 | 6 | 6 | 6 | 6 | 6 | 2 | 5 | 6 |
| Lorol^{®} C 18 | 20 | 20 | 20 | - | 20 | 20 | - | - | 20 |
| Lanette^{®} O | - | - | - | 20 | - | - | 10 | 12 | - |
| Eumulgin^{®} B 3 | 3 | 3 | 3 | 3 | 3 | 3 | 3 | 3 | - |
| Cutina^{®} E 24 PF | - | - | - | - | 5 | - | - | - | - |
| Aluminiumchlorohydrat | 20 | 20 | 20 | 20 | 20 | 20 | 20 | - | - |
| Talkum | 7,9 | 7 | 7,9 | 7,9 | 7,95 | 7,8 | 7,9 | 27 | 26,5 |
| isopuiegoi | 0,1 | - | - | - | - | - | - | - | - |
| Eugenylglucosid | - | 1,0 | - | - | - | - | - | - | - |
| Zinkgluconat | - | - | - | 0,1 | - | - | - | - | - |
| Zinkpyrrolidoncarbonsäure | - | - | - | - | 0,05 | - | - | - | - |
| Zinkcocoylethersulfat | - | - | - | - | - | 0,2 | - | - | - |
| Zinkglycinatmonohydrat | - | - | - | - | - | - | 0,1 | - | - |
| Ascorbylglucosid | - | - | - | - | - | - | - | 1,0 | - |
| 2-Ethylhexylglycerinether | - | - | - | - | - | - | - | - | 1,5 |
| Maltosin | 0,05 | 0,1 | 0,25 | 0,5 | 0,75 | 1,0 | 1,25 | 1,5 | 1,75 |
| Lactose | - | - | 0,05 | 0,05 | 0,03 | 0,04 | - | - | - |
| Glucose | - | - | 0,01 | - | - | - | - | - | - |
| Galactose | - | - | - | 0,02 | 0,01 | - | - | - | - |

Sprühfähige, translucente Antitranspirant-Mikroemulsionen (Angaben in Gew.-%)

| | 11.1 | 11.2 | 11.3 | 11.4 | 11.5 | 11.6 | 11.7 | 11.8 | 11.9 |
|---|---|---|---|---|---|---|---|---|---|
| Plantaren^{®} 1200 | 1,71 | 1,71 | - | 1,71 | 1,71 | - | 1,71 | 1,71 | 1,71 |
| Plantaren^{®} 2000 | 1,14 | 1,39 | 2,40 | 1,14 | 1,39 | 2,40 | 1,14 | 1,39 | 1,39 |
| Glycerinmonooleat | 0,71 | 0,71 | - | 0,71 | 0,71 | - | 0,71 | 0,71 | 0,71 |
| Dioctylether | 4,00 | 4,00 | 0,09 | 4,00 | 4,00 | 0,09 | 4,00 | 4,00 | 4,00 |
| Octyldodecanol | 1,00 | 1,00 | 0,02 | 1,00 | 1,00 | 0,02 | 1,00 | 1,00 | 1,00 |
| Parfümöl | 1,00 | 1,00 | 1,00 | 1,00 | 1,00 | 1,00 | 1,00 | 1,00 | 1,00 |
| Aluminiumchlorohydrat | 8,00 | 5,00 | 5,00 | - | - | - | 8,00 | 5,00 | 5,00 |
| 1,2-Propylenglycol | 5,00 | 5,00 | - | 5,00 | 5,00 | - | 5,00 | 5,00 | 5,00 |
| Glycerin | - | - | 5,00 | - | - | 5,00 | - | - | - |
| Phenoxyethanol | 1,0 | - | - | - | 1,0 | 1,0 | - | - | - |
| Zinklactat | - | 0,2 | - | - | - | - | - | - | - |
| Hinokitiol | - | - | 0,01 | - | - | - | - | - | - |
| Kupfergluconat | - | - | - | 0,1 | - | - | - | - | - |
| Glycin | - | - | - | - | - | - | 0,2 | - | - |
| Phytinsäure | - | - | - | - | - | - | - | 1,0 | - |
| Trikaliumcitrat | - | - | - | - | - | - | - | - | 1,0 |
| Maltosin | 1,5 | 2,0 | 1,2 | 1,0 | 0,5 | 0,6 | 0,7 | 0,8 | 0,9 |
| Lactose | - | - | 0,05 | 0,05 | 0,03 | 0,04 | - | - | - |
| Glucose | - | - | 0,01 | - | - | - | - | - | - |
| Galactose | - | - | - | 0,02 | 0,01 | - | - | - | - |
| Wasser | ad 100 | ad 100 | ad 100 | ad 100 | ad 100 | ad 100 | ad 100 | ad 100 | ad 100 |

Seifenhaltige Stifte (Angaben in Gew.-%)

| | 12.1 | 12.2 | 12.3 | 12.4 | 12.5 | 12.6 | 12.7 |
|---|---|---|---|---|---|---|---|
| Ethanol | 22,5 | 22,5 | 22,5 | 22,5 | 22,5 | 22,5 | 22,5 |
| Cutina^{®} FS 45 | 4,4 | 4,4 | 4,4 | 4,4 | 4,4 | 4,4 | 4,4 |
| 1,3 Butandiol | 31,7 | 31,7 | 31,7 | 31,7 | 31,7 | 31,7 | 31,7 |
| 1,2 Propylenglykol | 21,0 | 21,0 | 21,0 | 21,0 | 21,0 | 21,0 | 21,0 |
| Eutanol^{®} G | 2,0 | 2,0 | 2,0 | 2,0 | 2,0 | 2,0 | 2,0 |
| Aethoxal^{®} B | 3,0 | 3,0 | 3,0 | 3,0 | 3,0 | 3,0 | 3,0 |
| Cremophor^{®} RH 455 | 0,05 | 0,05 | 0,05 | 0,05 | 0,05 | 0,05 | 0,05 |
| NaOH 45 %ig | 1,44 | 1,44 | 1,44 | 1,44 | 1,44 | 1,44 | 1,44 |
| Phenoxyethanol | 1,0 | 1,0 | 1,0 | 1,0 | 1,0 | 1,0 | 1,0 |
| Sensiva^{®} SC 50 | 0,3 | 0,3 | 0,3 | 0,3 | 0,3 | 0,3 | 0,3 |
| Parfümöl | 2,0 | 2,0 | 2,0 | 2,0 | 2,0 | 2,0 | 2,0 |
| Eugenylglucosid | 1,0 | - | - | - | - | - | - |
| Dequest^{®} 2066 | - | - | - | 0,2 | - | - | - |
| Grüner Tee Extrakt | - | 0,015 | - | - | - | - | - |
| Maltosin | 0,1 | 0,5 | 0,15 | 0,25 | 1,0 | 0,4 | 1,5 |
| Lactose | - | - | - | 0,05 | 0,1 | - | - |
| Glucose | | | | 0,1 | | 0,02 | |
| Galactose | - | - | 0,01 | - | 0,03 | - | 0,2 |
| Phenylethylalkohol | - | - | - | - | - | - | 1,0 |
| Wasser dest. | ad 100 | ad 100 | ad 100 | ad 100 | ad 100 | ad 100 | ad 100 |

Deodorant im Pumpzerstäuber (Angaben in Gew.-%)

| | 13.1 | 13.2 | 13.3 | 13.4 | 13.5 | 13.6 | 13.7 | 13.8 |
|---|---|---|---|---|---|---|---|---|
| Ethanol 96 %-ig, (DEP vergällt) | 55,0 | 55,0 | 55,0 | 55,0 | 55,0 | 55,0 | 55,0 | 55,0 |
| Triethylcitrat | 3,0 | 3,0 | 3,0 | 3,0 | 3,0 | 3,0 | 3,0 | 3,0 |
| Cremophor^{®} RH 455 | 0,5 | 0,5 | 0,5 | 0,5 | 0,5 | 0,5 | 0,5 | 0,5 |
| Eucarol^{®} AGE-EC | 1,0 | - | - | - | - | - | - | - |
| Eucarol^{®} AGE-ET | - | 0,2 | - | - | - | - | - | - |
| Eucarol^{®} AGE-SS | - | - | 1,0 | - | - | - | - | - |
| Dequest^{®} 2016 D | - | - | - | - | 0,1 | - | - | - |
| Maltosin | 0,1 | 0,5 | 0,15 | 0,25 | 1,0 | 0,4 | 1,5 | 2,0 |
| Lactose | - | - | 0,05 | 0,05 | 0,03 | 0,04 | - | - |
| Glucose | - | - | 0,01 | - | - | - | - | - |
| Galactose | - | - | - | 0,02 | 0,01 | - | - | - |
| Parfümöl | 1,0 | 1,0 | 1,0 | 1,0 | 1,0 | 1,0 | 1,0 | 1,0 |
| Wasser | ad 100 | ad 100 | ad 100 | ad 100 | ad 100 | ad 100 | ad 100 | ad 100 |

Wasserfreies Deodorant-Spray (Angaben in Gew.-%)

| | 14.1 | 14.2 |
|---|---|---|
| 2-Octyldodecanol | 0,5 | 0,5 |
| Ethanol 99 %-ig, (DEP vergällt) | 39 | 39,45 |
| Naringin | 0,05 | |
| Hinokitiol | - | 0,01 |
| Maltosin | 0,1 | 0,5 |
| n-Butan | ad 100 | ad 100 |

Antitranspirant Roll-on (Angaben in Gew.-%)

| | 15.1 | 15.2 | 15.3 |
|---|---|---|---|
| Ethanol 96 %-ig,(DEP vergällt) | 30,0 | 30,0 | 30,0 |
| Mergital^{®} CS 11 | 2,0 | 2,0 | 2,0 |
| Eumulgin^{®} B 3 | 2,0 | 2,0 | 2,0 |
| Aluminiumchlorohydrat | 20,0 | 20,0 | 20,0 |
| Hydroxyethylcellulose | 0,5 | 0,5 | 0,5 |
| Dermosoft^{®} HMA | 0,5 | - | - |
| Maltosin | 0,1 | 0,5 | 0,15 |
| Parfümöl | 0,8 | 0,8 | 0,8 |
| Wasser | ad 100 | ad 100 | ad 100 |

Antitranspirant-Spray vom Suspensionstyp (Angaben in Gew.-%)

| | 16.1 | 16.2 | 16.3 | 16.4 | 16.5 |
|---|---|---|---|---|---|
| DC-245 | 10,0 | 10,0 | 10,0 | 10,0 | 10,0 |
| Isopropylmyristat | 5,0 | 5,0 | 5,0 | 5,0 | 5,0 |
| Aluminiumchlorohydrat-Pulver | 5,0 | 5,0 | 5,0 | 5,0 | 5,0 |
| Aerosil^{®} R 972 | 2,0 | 2,0 | 2,0 | 2,0 | 2,0 |
| Geraniol-7 EO | 0,5 | - | 0,5 | - | 0,5 |
| Alutrat® | - | 2,0 | - | 2,0 | - |
| Maltosin | 0,1 | 0,5 | 0,15 | 0,25 | 1,0 |
| Lactose | - | - | 0,05 | 0,1 | - |
| Glucose | - | - | 0,1 | - | 0,02 |
| Galactose | - | 0,03 | 0,02 | - | - |
| n-Butan | ad 100 | ad 100 | ad 100 | ad 100 | ad 100 |

### Antitranspirant-Tücher (Beispiele Nr. 17.1. - 17.4)

Für die erfindungsgemäße Ausführungsform als Antitranspirant-Tuch wurde ein einlagiges Substrat aus 100 % Viskose mit einem Flächengewicht von 50 g/m² mit jeweils 75 g der Beispielemulsionen 11.1 bzw. 11.2 bzw. 2.3 pro Quadratmeter oder mit jeweils 75 g der Beispiellösungen 13.1 bzw. 13.2 beaufschlagt, in Tücher geeigneter Größe geschnitten und in Sachets verpackt.

Wasser-in-Öl-Emulsionssprays

| Bestandteil | Beispiel 18.1 [Gew.-%, bezogen auf Gesamtzubereitung incl. Treibgas] | Beispiel 18.1 [Gew.-%, bezogen auf W/O-Emulsion] | Beispiel 18.2 [Gew.-%, bezogen auf Gesamtzube-reitung incl. Treibgas] | Beispiel 18.2 [Gew.-%, bezogen auf W/O-Emulsion] |
|---|---|---|---|---|
| Dow Corning 345 Fluid | 2,0 | 12,5 | 1,0 | 4,9 |
| 1,2-Propylenglycol | 0,5 | 3,1 | 2,0 | 9,8 |
| Dow Corning 5225 C | 3,0 | 16,9 (Öl) | 2,5 | 11,0 (Öl) |
| | | 1,9 (Emulgator) | | 1,2 (Emulgator) |
| 2-Ethylhexylpalmitat | 0,5 | 3,1 | 0,5 | 2,4 |
| Phenoxyethanol | 0,08 | 0,5 | 0,1 | 0,5 |
| Maltosin | 1,0 | 0,5 | 1,5 | 0,25 |
| Wasser, vollentsalzt | 4,904 | 30,6 | 7,14 | 34,85 |
| Microdry | 5,0 | 31,3 | 7,25 | 35,3 |
| n-Butan | 84,0 | - | 79,5 | - |

W/O-Emulsions-Kompaktspray

| Bestandteil | Beispiel 19.1 [Gew.-%, bezogen auf Gesamtzubereitung incl. Treibgas] | Beispiel 19.1 [Gew.-%, bezogen auf W/O-Emulsion] | Beispiel 19.2 [Gew.-%, bezogen auf Gesamtzubereitung incl. Treibgas] | Beispiel 19.2 [Gew.-%, bezogen auf W/O-Emulsion] |
|---|---|---|---|---|
| Dow Corning 345 Fluid | 0,48 | 1,6 | 2,173 | 4,1 |
| 1,2-Propylenglycol | 1,5 | 5,0 | 4,823 | 9,1 |
| Dow Corning 5225 C | 4,2 | 12,53 (Cyclomethicone) 1,47(Emulgator) | 7,42 | 12,53 (Cyclomethicone) 1,47(Emulgator) |
| Benzoesäure-C12-15-alkylester | 1,38 | 4,6 | - | - |
| 2-Ethylhexylpalmitat | - | - | 1,192 | 2,25 |
| Parfum | 0,75 | 2,5 | - | - |
| Phenoxyethanol | 0,15 | 0,5 | 0,265 | 0,5 |
| Maltosin | 0,1 | 0,5 | 0,15 | 0,25 |
| Wasser, vollentsalzt | 1,71 | 5,7 | 2,12 | 4,0 |
| Aluminiumchlorohydrat, 50%ige wässrige Lösung | 19,8 | 66,0 | 34,98 | 66,0 |
| n-Butan | 70,0 | - | 47,0 | - |

Die erfindungsgemäßen Beispielzusammensetzungen werden auf die Haut, insbesondere die Achselhaut, aufgetragen.

Die erfindungsgemäßen Zusammensetzungen zeichnen sich durch eine hohe, gegenüber dem Stand der Technik verbesserte Hautverträglichkeit bei gleichzeitig hoher Inhibierung des Haarwuchses aus.

Liste der verwendeten Rohstoffe

| Komponente | INCI | Lieferant/Hersteller |
|---|---|---|
| Aethoxal^{®} B | PPG-5-Laureth-5 | Cognis |
| Alutrat^{®} | Aluminiumcitrat | Vevy |
| Cetiol^{®} OE | DICAPRYLYL ETHER | Cognis |

| Cremophor^{®} RH 455 | hydriertes Ricinusöl mit 40 EO, Propylenglykol-haltig | BASF |
|---|---|---|
| Cutina ^{®} AGS | Glycol Distearate | Cognis |
| Cutina ^{®} EGMS | Glycol Stearate | Cognis |
| Cutina ^{®} PES | Pentaerythrityl Distearate | Cognis |
| Cutina^{®} CP | Cetyl Palmitate | Cognis |
| Cutina^{®} FS45 | Palmitic Acid, Stearic Acid | Cognis |
| Cutina^{®} HR | Hydrogenated Castor Oil | Cognis |
| Cutina^{®} E 24 PF | PEG-20 GLYCERYL STEARATE | Cognis |
| DC^{®} 245 | Cyclopentasiloxan | Dow Corning |
| Dequest^{®} 2016 D | 1-Hydroxyethan-1,1-diphosphonsäure-tetra-Natrium-Salz, Aktivsubstanz 84 % | Solutia (Monsanto) |
| Dequest^{®} 2066 | Diethylentriaminpenta(methylenphosp honsäure)-Natriumsatz, Aktivsäure 45 - 49 % | Solutia (Monsanto) |
| Dermosoft HMA | p-Hydroxymandelsäure Natriumsalz, Aktivsubstanz 95 - 96 % | Dr. Straetmans |
| Dow Corning 200 Fluid 5 cst | Dimethicone | Dow Corning |
| Dow Corning 345 Fluid | Cyclomethicone (Decamethylcyclopentasiloxan, Dodecamethylcyclohexasiloxan) | Dow Corning |
| Dow Corning 5225 C Formulation Aid | Cyclomethicone, PEG/PPG-18/18 Dimethicone im Gewichtsverhältnis 9:1 | Dow Corning |
| Eucarol^{®} AGE-EC-UP | Disodium Cocopolyglucose Citrate, 30 % Aktivsubstanz in Wasser | Cesalpinia Chemicals |
| Eucarol^{®} AGE-ET-UP | Disodium Cocopolyglucose Tartrate, 30 % Aktivsubstanz in Wasser | Cesalpinia Chemicals |
| Eucarol^{®} AGE-SS | Disodium Cocopolyglucose Sulfosuccinate, 45 % Aktivsubstanz in Wasser | Cesalpinia Chemicals |
| Eumulgin^{®} B2 | Ceteareth-20 | Cognis |
| Eumulgin^{®} B3 | Ceteareth-30 | Cognis |
| Eumulgin^{®} B 3 | CETEARETH-30 | Cognis |
| Eutanol^{®} G | Octyldodecanol | Cognis |
| Eutanol^{®} G 16 | 2-Hexyldecanol | Cognis |
| Grüner Tee Extrakt | pulverförmig | Dragoco |
| Kesterwachs K62 | Cetearyl Behenate | Koster Keunen |
| Lanette^{®} O | Cetyl-/Stearylalkohol im Verhältnis 1:1 | Cognis |
| Locron L (ACH-Lösung 50%ig) | Aluminum Chlorohydrate | Clariant |
| Lorol^{®} C 18 | Stearylalkohol | Cognis |
| Mergital^{®} CS 11 | CETEARETH-11 | Cognis |
| Microdry^{®} | Aluminium Chlorohydrate | Reheis |
| Novata^{®} AB | Cocoglycerides | Cognis |
| Plantaren^{®} 1200 | LAURYL GLUCOSIDE, ca. 50 % Aktivsubstanz | Cognis |
| Plantaren^{®} 2000 | DECYL GLUCOSIDE, ca. 50 % Aktivsubstanz | Cognis |
| Sensiva^{®} SC 50 | 2-Ethylhexylglycerinether | Schülke & Mayr |
| Tinogard AS | Bumetrizole | Ciba |
| Tinogard Q | Tris(tetramethyl-hydroxypiperidinol)citrate | Ciba |
| Trilon A | Nitrilotriessigsäure 3 Na | BASF |
| Trilon B | EDTA-4 Na | BASF |
| Trilon^{®} M | Methylglycindiessigsäure-tri-NatriumSalz, Aktivsubstanz 39 - 41 % | BASF |
| Ucon Fluid^{®} AP | PPG-14 BUTYL ETHER | Amerchol (Union Carbide) |

## Patentansprüche

1. Kosmetische oder dermatologische Deodorant- oder Antitranspirant-Zusammensetzung, enthaltend in einem kosmetisch oder dermatologisch verträglichen Träger
a) mindestens einen Deodorant- oder Antitranspirant-Wirkstoff,
b) mindestens eine Verbindung aus der Gruppe der Advanced Glycation Endproducts (AGEs) und/oder deren Vorläufern, ausgewahlt aus den Verbindungen gemäß Formel 15 und 15a.

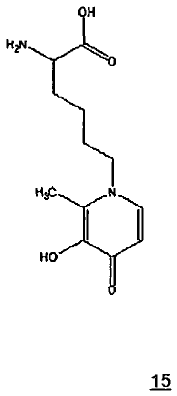

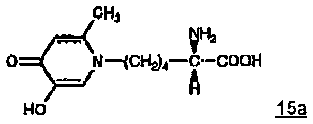

2. Deodorant- oder Antitranspirant-Zusammensetzung nach Anspruch 1, **dadurch gekennzeichnet, dass** sie - bezogen auf das Gewicht der anwendungsbereiten Zusammensetzung - 0,00001 bis 15 Gel.-%, vorzugsweise 0,0001 bis 10 Gew.-%, besonders bevorzugt 0,001 bis 6 Gew.-%, weiter bevorzugt 0,01 bis 4 Gew.-% und insbesondere 0,1 bis 2 Gel.-% Advanced Glycation Endproducts (AGEs) und/oder deren Vorläufer enthält.

3. Deodorant- oder Antitranspirant-Zusammensetzung nach einem der Ansprüche 1 oder 2, **dadurch gekennzeichnet, dass** sie sie - bezogen auf das Gewicht der anwendungsbereiten Zusammensetzung - 0,0001 bis 15 Gew.-%, vorzugsweise 0,001 bis 12,6 Gew.-%, besonders bevorzugt 0,01 bis 10 Gew.-%, weiter bevorzugt 0,1 bis 5 Gew.-% und Insbesondere 0,25 bis 2,5 Gew.-% Maltosin (Formel 15) enthält.

4. Deodorant- oder Antitranspirant-Zusammensetzung nach einem der Ansprüche 1 bis 3, **dadurch gekennzeichnet, dass** der mindestens eine Antitranspirant-Wirkstoff in einer Gesamtmenge von 3 - 25 Gew.-%, vorzugsweise 5 - 22 Gew.-% und insbesondere 10 - 20 Gew.-%, bezogen auf das Gesamtgewicht der Aktivsubstanz in der gesamten Zusammensetzung, enthalten ist.

5. Deodorant- oder Antitranspirant-Zusammensetzung nach einem der Ansprüche 1 bis 4, **dadurch gekennzeichnet, dass** der mindestens eine Deodorant-Wirkstoff in einer Gesamtmenge von 0,1 -10 Gew.-%, bevorzugt 0,2 - 7 Gew.-%, besonders bevorzugt 0,3 - 5 Gew.-% und außerordentlich bevorzugt 0,4-1,0 Gew.-%, jeweils bezogen auf das Gesamtgewicht der Aktivsubstanz des Deodorant-Wirkstoffs oder der Deodorant-Wirkstoffe in der Gesamtzusammensetzung, enthalten ist.

6. Deodorant- oder Antitranspirant-Zusammensetzung nach einem der Ansprüche 1 bis 5, **dadurch gekennzeichnet, dass** der kosmetisch oder dermatologisch verträgliche Träger als Puder, In Stiftform, als Aerosolspray, Pumpspray, flüssige oder gelförmige Roll-on-Applikation, Creme, Lotion, Lösung, Gel oder auf einem Substrat aufgebracht vorliegt

7. Deodorant- oder Antitranspirant-Zusammensetzung nach einem der Ansprüche 1 bis 6, **dadurch gekennzeichnet, dass** der mindestens eine Deodorant-Wirkstoff ausgewählt ist aus Arylsulfatase-Inhibitoren, β-Glucuronidase-Inhibitoren, Aminoacylase-Inhibitoren, Esterase-Inhibitoren, Lipase-Inhibitoren und Lipoxigenase-Inhibitoren, alpha-Monoalkylglycerinethern mit einem verzweigten oder linearen gesättigten oder ungesättigten, gegebenenfalls hydroxylierten C₆ - C₂₂-Alkylrest, Phenoxyethanol, keimhemmend wirkenden Parfümölen, präbiotisch wirksamen Komponenten, Trialkylcitronensäureestern, Wirkstoffen, die die Zahl der an der Geruchsbildung betelligten Hautkelme aus der Gruppe der Staphylokokken, Corynebakterien, Anaerokokken und Mikrokokken reduzieren bzw. deren Wachstum hemmen, Zinkverbindungen, Organohalogenverbindungen, quartären Ammoniumverbindungen, Geruchsabsorbern, Natriumbicarbonat, Lantibiotika, sowie Mischungen der vorgenannten Substanzen.

8. Deodorant- oder Antitranspirant-Zusammensetzung nach einem der Ansprüche 1 bis 7, **dadurch gekennzeichnet, dass** sowohl mindestens ein Deodorant-Wirkstoff als auch mindestens ein Antitranspirant-Wirkstoff enthalten sind.

9. Deodorant- oder Antitranspirant-Zusammensetzung nach einem der Ansprüche 1 bis 8, **dadurch gekennzeichnet, dass** weiterhin mindestens eine Duftstoffkomponente enthalten ist.

10. Nicht-Therapeutisches Verfahren zur Verringerung des Haarwuchses, wobei eine Deodorant- oder Antitranspirant-Zusammensetzung nach einem der Ansprüche 1 bis 9 topisch in einer wirksamen Menge auf die Haut, insbesondere auf die Haut im Achselbereich, aufgetragen wird.

11. Nicht-Therapeutisches Verfahren zur Behandlung von Körpergeruch, **dadurch gekennzeichnet, dass** eine Zubereitung nach einem der Ansprüche 1 bis 9 auf die Haut aufgetragen wird.

## Claims

1. Cosmetic or dermatologic deodorant or antiperspirant composition, comprising in a cosmetic or dermatological compatible carrier
a) at least one deodorant or antiperspirant active agent,
b) at least one compound from the group of the Advanced Glycation End-products (AGEs) and/or their precursors, selected from the compounds according to Formula 15 and 15a.

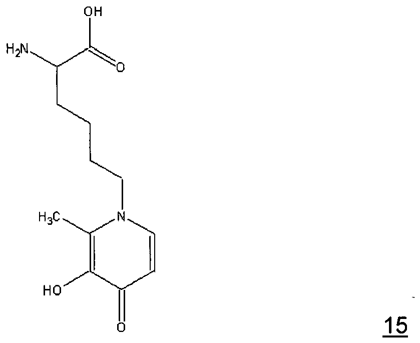

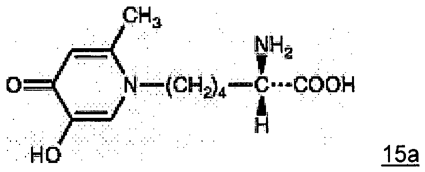

2. Deodorant or antiperspirant composition according to claim 1, **characterised in that** it comprises - based on the weight of the ready for use composition - 0.00001 to 15 wt %, preferably 0.0001 to 10 wt %, particularly preferably 0.001 to 5 wt %, more preferably 0.01 to 4 wt % and in particular 0.1 to 2 wt % Advanced Glycation End-products (AGEs) and/or their precursors.

3. Deodorant or antiperspirant composition according to one of claims 1 or 2, **characterised in that** it comprises - based on the weight of the ready for use composition - 0.0001 to 15 wt %, preferably 0.001 to 12.5 wt %, particularly preferably 0.01 to 10 wt %, more preferably 0.1 to 5 wt % and in particular 0.25 to 2.5 wt % maltosin (Formula 15).

4. Deodorant or antiperspirant composition according to one of claims 1 to 3, **characterised in that** the at least one antiperspirant active agent is comprised in a total amount of 3 - 25 wt %, preferably 5 - 22 wt % and in particular 10 - 20 wt %, based on the total weight of the active substance in the total composition.

5. Deodorant or antiperspirant composition according to one of claims 1 to 4, **characterised in that** the at least one deodorant active agent is comprised in a total amount of 0.1 - 10 wt %, preferably 0.2 - 7 wt %, particularly preferably 0.3 - 5 wt % and exceptionally preferably 0.4 - 1.0 wt %, each based on the total weight of the active substance of the deodorant active agent or of the deodorant active agents in the total composition.

6. Deodorant or antiperspirant composition according to one of claims 1 to 5, **characterised in that** the cosmetic or dermatological compatible carrier is present as a powder, in the form of a pen, as an aerosol spray, pump spray, liquid or gel roll-on application, cream, lotion, solution, gel or deposited on a substrate.

7. Deodorant or antiperspirant composition according to one of claims 1 to 6, **characterised in that** the at least one deodorant active agent is selected from arylsulfatase inhibitors, β-glucuronidase inhibitors, aminoacylase inhibitors, esterase inhibitors, lipase inhibitors and lipoxigenase inhibitors, alpha-monoalkyl glycerine ethers with a branched or linear saturated or unsaturated, optionally hydroxylated C₆ - C₂₂ alkyl group, phenoxyethanol, germ-inhibiting perfume oils, prebiotic active components, trialkyl esters of citric acid, active agents that reduce the number or inhibit the growth of skin germs from the group of the staphylococci, corynebacteria, anaerococci and micrococci involved in the formation of odours, zinc compounds, organohalogen compounds, quaternary ammonium compounds, odour absorbers, sodium bicarbonate, Lantibiotics, as well as mixtures of the above cited substances.

8. Deodorant or antiperspirant composition according to one of claims 1 to 7, **characterised in that** at least one deodorant active agent as well as at least one antiperspirant active agent are both comprised.

9. Deodorant or antiperspirant composition according to one of claims 1 to 8, **characterised in that** at least one scent component is additionally comprised.

10. Non-therapeutic method for reducing hair growth, wherein an effective amount of a deodorant or antiperspirant composition according to one of claims 1 to 9 is topically applied onto the skin, in particular onto the skin in the axillary region.

11. Non-therapeutic method for the treatment of body odour, **characterised in that** a preparation according to one of claims 1 to 9 is applied onto the skin.

## Revendications

1. Composition cosmétique ou dermatologique de déodorant ou d'antitranspirant contenant, dans un support compatible du point de vue cosmétique ou du point de vue dermatologique :
a) au moins une substance active déodorante ou antitranspirante ;
b) au moins un composé du groupe des Advanced Glycation Endproducts (AGE) et/ou de leurs précurseurs, choisi parmi les composés répondant aux formules 15 et 15a

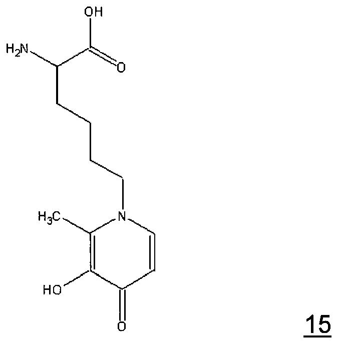

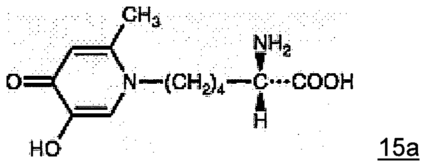

2. Composition de déodorant ou d'antitranspirant selon la revendication 1, **caractérisée en ce qu'**elle contient - rapportés au poids de la composition prête à l'emploi - de 0,00001 à 15 % en poids, de préférence de 0,0001 à 10 % en poids, de manière particulièrement préférée de 0,001 à 5 % en poids, de manière plus préférée de 0,01 à 4 % en poids et en particulier de 0,1 à 2 % en poids des Advanced Glycation Endproducts (AGE) et/ou de leurs précurseurs.

3. Composition de déodorant ou d'antitranspirant selon la revendication 1 ou 2, **caractérisée en ce qu'**elle contient - rapportés au poids de la composition prête à l'emploi - de 0,0001 à 15 % en poids, de préférence de 0,001 à 12,5 % en poids, de manière particulièrement préférée de 0,01 à 10 % en poids, de manière plus préférée de 0,1 à 5 % en poids et en particulier de 0,25 à 2,5 % en poids de maltosine (formule 15).

4. Composition de déodorant ou d'antitranspirant selon l'une quelconque des revendications 1 à 3, **caractérisée en ce que** ladite au moins une substance active antitranspirante est contenue en une quantité totale de 3 à 25 % en poids, de préférence de 5 à 22 % en poids et en particulier de 10 à 20 % en poids, rapportés au poids total de la substance active dans la composition totale.

5. Composition de déodorant ou d'antitranspirant selon l'une quelconque des revendications 1 à 4, **caractérisée en ce que** ladite au moins une substance active déodorante est contenue en une quantité totale de 0,1 à 10 % en poids, de préférence de 0,2 à 7 % en poids et en particulier de 0,3 à 5 % en poids et de manière extraordinairement préférée de 0,4 à 1,0 % en poids, chaque fois rapportés au poids total de la substance active déodorante ou des substances actives déodorantes dans la composition totale.

6. Composition de déodorant ou d'antitranspirant selon l'une quelconque des revendications 1 à 5, **caractérisée en ce que** le support compatible du point de vue cosmétique ou du point de vue dermatologique est présent sous la forme d'une poudre, sous la forme d'un bâton, sous la forme d'un spray aérosol, d'un spray à pompe, d'une application liquide ou géliforme à appliquer par roulement, d'une crème, d'une lotion, d'une solution, d'un gel ou à l'état appliqué sur un substrat.

7. Composition de déodorant ou d'antitranspirant selon l'une quelconque des revendications 1 à 6, **caractérisée en ce que** ladite au moins une substance active déodorante est choisie parmi des inhibiteurs d'arylsulfatase, des inhibiteurs de β-glucuronidase, des inhibiteurs d'aminoacylase, des inhibiteurs d'estérase, des inhibiteurs de lipase et des inhibiteurs de lipoxygénase, des éthers d'alpha-monoalkylglycérol comprenant un résidu alkyle en C₆-C₂₂ linéaire ou ramifié, saturé ou insaturé, facultativement hydroxylé, le phénoxyéthanol, des huiles parfumées bactériostatiques, des composants à activité prébiotique, des esters d'acide trialkylcitrique, des substances actives qui réduisent le nombre des germes cutanés impliqués dans l'apparition d'odeurs, choisi parmi le groupe des staphylocoques, des corynebactéries, des anaérocoques et des microcoques, respectivement qui inhibent leur croissance, des composés du zinc, des composés organohalogénés, des composés d'ammonium quaternaires, des absorbeurs de l'odeur, le bicarbonate de sodium, des lantibiotiques, ainsi que des mélanges des substances susmentionnées.

8. Composition de déodorant ou d'antitranspirant selon l'une quelconque des revendications 1 à 7, **caractérisée en ce qu'**elle contient aussi bien au moins une substance déodorante qu'au moins une substance antitranspirante.

9. Composition de déodorant ou d'antitranspirant selon l'une quelconque des revendications 1 à 8, **caractérisée en ce qu'**elle contient en outre au moins un composant parfumé.

10. Procédé non thérapeutique pour la réduction de la croissance pileuse, dans lequel on applique une composition de déodorant ou d'antitranspirant selon l'une quelconque des revendications 1 à 9, par voie locale en une quantité efficace sur la peau, en particulier sur la peau dans la zone des aisselles.

11. Procédé non thérapeutique pour le traitement de l'odeur corporelle, **caractérisé en ce qu'**on applique une préparation selon l'une quelconque des revendications 1 à 9, sur la peau.
